# EUROPEAN PATENT APPLICATION

(11) **EP 3 722 311 A1**
(43) Date of publication of application: **14.10.2020**
(21) Application number: 18886699.0
(22) Date of filing: 05.12.2018
(51) Int. Cl.: C07K 16/24, C12N 15/13, A61K 39/395, A61P 35/00

(54) **ANTI-HUMAN IL6 MONOCLONAL ANTIBODIES, PREPARATION METHOD THEREFOR AND USE THEREOF**

(30) Priority: 05.12.2017 CN 201711269749
(71) Applicant: Nanjingjinsirui Science&Technology Biology Corp., Nanjing, Jiangsu 211100 (CN)
(72) Inventor: YIN, Liusong, Nanjing Jiangsu 211100 (CN); LIN, Feng, Nanjing Jiangsu 211100 (CN); WANG, Lei, Nanjing Jiangsu 211100 (CN); XU, Yunyun, Nanjing Jiangsu 211100 (CN); DING, Li, Nanjing Jiangsu 211100 (CN)
(74) Representative: Pharma Patents International AG
(86) International application number: PCT/CN2018/119353
(87) International publication number: WO 2019/109947

(57) **Abstract**

The present invention provide anti-human IL6 monoclonal antibodies, amino acid sequences of the variable region of the heavy chain and of the variable region of the light chain thereof, and encoding nucleotide sequences thereof. The present invention also provides a method for preparing the anti-human IL6 monoclonal antibodies and use of the anti-human IL6 monoclonal antibodies in the preparation of an antitumor drug. The present anti-human IL6 monoclonal antibodies can inhibit cell proliferation by blocking an IL6 signal pathway, thereby achieving the purpose of tumor immunotherapy.

## Description

### Field of The Invention

The present invention belongs to the field of tumor immunotherapy and molecular immunology, particularly relates to anti-human IL6 monoclonal antibodies. The present invention further relates to a method for preparing the anti-human IL6 monoclonal antibodies, and use of the anti-human IL6 monoclonal antibodies.

### Background of The Invention

Immune system is a host defense system. In order to function normally, an immune system must be able to detect the invasion of foreign pathogens sensitively, and distinguish them from the healthy tissues of an organism itself. The immune system of a vertebrate is a functional system consisting of a variety of organs, tissues, cells and molecules, and is the most effective mechanism for an organism to defend against the invasion of foreign substances; these immune organs, tissues, cells and molecules cooperate with each other and restrict each other to achieve a balance, so as to protect the organism from external infestation and maintain the balance in the body (Janeway et al., Immunology: The Immune System in Health and Disease. New York: Garland Science, 2005). Such mutual cooperation and mutual restriction require the coordination of numerous immune checkpoint proteins and cytokines, wherein stimulatory immune checkpoint proteins enhance the defense response of an immune system, and inhibitory immune checkpoint proteins control an overly strong immune system to prevent the generation of an autoimmune response. Carcinogenesis is resulted from the loss of normal cell regulatory functions in normal somatic cells and the accumulation of gene mutations to a certain degree. In tumor treatment, tumor immunotherapy is widely used, and has become one of the most important means for tumor treatment. Tumor immunotherapy enables human T cells to better recognize cancerous cells by enhancing the immune function in human body, thereby killing cancer cells. For example, Pembrolizumab, an antibody against inhibitory immune checkpoint protein PD1, and Ipilimumab, an antibody against CTLA4, have been approved for use in the treatment of a variety of cancer indications. However, due to tumor heterogeneity and inter-tumor differences of tumor immunity, these immune checkpoint proteins-based antibody drugs have very limited clinical benefits.

Interleukins, the most important category of cytokines, have a variety of immunomodulary functions of directing the maturation, differentiation, migration and adherence of cells in immune system. In tumorigenesis, these cytokines directly stimulate immune effectors and stromal cells in tumor sites and enhance tumor cell recognition of cytotoxic effector cells (Yoshimoto et al., Immunotherapy 5:825-844, 2009). Recently, some studies show that interleukins participate in a lot of tumor-associated molecule mechanisms and have been used to develop many cytokine-based cancer treatment methods. However, tumor cells can escape from immune system by many methods, including establishment of a safe tumor microenvironment, secretion of cytokines and anti-inflammatory cytokines that suppress immune system, and recruitment or conversion of inflammatory cells that suppress immune responses, including regulatory T (Treg) cells, bone marrow derived suppressor cells (MDSC) and dendritic cells (DC) (Stewart et al., Cancer Metastasis Rev. 30:125-140, 2011). Interleukin family members are present in the tumor microenvironment and interact with various biomolecules such as cancer stem cells, microRNAs, and epithelial mesenchymal cells. Therefore, based on the principle that interleukins affect mechanisms of tumor formation, high-efficient cancer immunotherapy can be developed. Over the past decade, some relevant therapies including interleukin 2 (IL2), interleukin 6 (IL6), interleukin 7 (IL7), interleukin 12 (IL12), interleukin 18 (IL18) and interleukin 21 (IL21) have entered the clinical trial stage in patients.

Interleukin 6 (IL6) has a variety of functions *in vivo,* among which this factor can cause imbalance and secretion of a variety of inflammatory substances of regulatory T cells (Treg). Studies have shown that evaluated serum IL6 concentration in patients is closely associated with tumor staging of various cancers (e.g., multiple myeloma, non-small cell lung cancer, colorectal cancer, renal cancer, prostate cancer, breast cancer and ovarian cancer) and survival of patients. Moreover, IL6 can induce epithelial-mesenchymal transition in breast cancer cells to generate CD44 positive cells with the characteristics of stem cells. There is evidence showing that the IL6 level in tumor-associated endothelial cells is directly associated with the tumorigenicity of tumor stem cells (Int. J. Mol. Sci. 16:1691-1710, 2015). Therefore, it is a potential treatment strategy for cancer to block IL6 signaling. The anti-human IL6 antibody Siltuximab from Johnson & Johnson Pharmaceuticals was approved by the U.S. Food and Drug Administration in 2014 for use in the treatment of Castleman's disease and now is undergoing a Phase II clinical trial directed to multiple myeloma. However, so far, there are no anti-human IL6 antibodies that are commercially available for tumor immunotherapy. Moreover, different anti-human IL6 monoclonal antibodies have different degrees of side effects, including the possibility of inducing immunogenicity in some patients, and different IL6 monoclonal antibodies have different degrees of developability. Therefore, there is still a need to develop new functional antibodies capable of blocking IL6 signaling, which should have higher affinity, specificity, functionality, and diversity.

### Summary of The Invention

In one aspect, the present invention provides an anti-human IL6 monoclonal antibody, comprising a heavy chain variable region and a light chain variable region, wherein the heavy chain variable region comprises HCDR1, HCDR2 and HCDR3, and the light chain variable region comprises LCDR1, LCDR2 and LCDR3, wherein the HCDR1, HCDR2, HCDR3, LCDR1, LCDR2 and LCDR3 are selected from one of the following combinations:
(a) the amino acid sequence of HCDR1 is RYWMH;
   the amino acid sequence of HCDR2 is YINPITGYTENNQKFKD;
   the amino acid sequence of HCDR3 is GIRGFTY;
   the amino acid sequence of LCDR1 is RASENVDNSDNSFMH;
   the amino acid sequence of LCDR2 is RASNLDS;
   the amino acid sequence of LCDR3 is QQTNEAPLT;
(b) the amino acid sequence of HCDR1 is NYWMH;
   the amino acid sequence of HCDR2 is YVIPSTGYTDYNQSFKD;
   the amino acid sequence of HCDR3 is LLPGFAY;
   the amino acid sequence of LCDR1 is RSSQSLVDSNGNTYLH;
   the amino acid sequence of LCDR2 is KVSNRFS;
   the amino acid sequence of LCDR3 is SQSTHVPPT;
(c) the amino acid sequence of HCDR1 is NYWMH;
   the amino acid sequence of HCDR2 is YIDPRTASIYYNQKFKD;
   the amino acid sequence of HCDR3 is ILYGKYDV;
   the amino acid sequence of LCDR1 is RSSQSLVDSNGNTYLH;
   the amino acid sequence of LCDR2 is KVSNRFS;
   the amino acid sequence of LCDR3 is SQSTHVPPT;
(d) the amino acid sequence of HCDR1 is DAWMD;
   the amino acid sequence of HCDR2 is EIRSKTYHPATYYTKSVRG;
   the amino acid sequence of HCDR3 is PRYYGGYFDY;
   the amino acid sequence of LCDR1 is RASESVDNYGMSFMN;
   the amino acid sequence of LCDR2 is TASNQGS;
   the amino acid sequence of LCDR3 is QQSKEVPYT;
(e) the amino acid sequence of HCDR1 is NYIIH;
   the amino acid sequence of HCDR2 is AIYPGNGDTSYSQKFKD;
   the amino acid sequence of HCDR3 is GDAGYSAWFAY;
   the amino acid sequence of LCDR1 is SASESVDSYGNNFMH;
   the amino acid sequence of LCDR2 is LASKLES;
   the amino acid sequence of LCDR3 is QQNNEDPLT;
(f) the amino acid sequence of HCDR1 is SHTVS;
   the amino acid sequence of HCDR2 is KMWSNGDTDYDSAIRS;
   the amino acid sequence of HCDR3 is YYFSSYGGGYFDY;
   the amino acid sequence of LCDR1 is RASKSVSTYMH;
   the amino acid sequence of LCDR2 is SASNLES;
   the amino acid sequence of LCDR3 is QQSDELPDT; and
(g) the amino acid sequence of HCDR1 is SFPMA;
   the amino acid sequence of HCDR2 is TISPSGGTSYSRDSVKG;
   the amino acid sequence of HCDR3 is ERIYNTYFDY;
   the amino acid sequence of LCDR1 is LPSEDISSDLA;
   the amino acid sequence of LCDR2 is NANTLPN;
   the amino acid sequence of LCDR3 is QQYDSYPYT.

In more particular embodiments, the amino acid sequences of the heavy chain variable region and light chain variable region of the anti-human IL6 monoclonal antibody are selected from one of the following combinations:
the heavy chain variable region comprising the amino acid sequence set forth in SEQ ID NO: 1, and the light chain variable region comprising the amino acid sequence set forth in SEQ ID NO: 3;
the heavy chain variable region comprising the amino acid sequence set forth in SEQ ID NO: 5, and the light chain variable region comprising the amino acid sequence set forth in SEQ ID NO: 7;
the heavy chain variable region comprising the amino acid sequence set forth in SEQ ID NO: 9, and the light chain variable region comprising the amino acid sequence set forth in SEQ ID NO: 11;
the heavy chain variable region comprising the amino acid sequence set forth in SEQ ID NO: 13, and the light chain variable region comprising the amino acid sequence set forth in SEQ ID NO: 15;
the heavy chain variable region comprising the amino acid sequence set forth in SEQ ID NO: 17, and the light chain variable region comprising the amino acid sequence set forth in SEQ ID NO: 19;
the heavy chain variable region comprising the amino acid sequence set forth in SEQ ID NO: 21, and the light chain variable region comprising the amino acid sequence set forth in SEQ ID NO: 23; and
the heavy chain variable region comprising the amino acid sequence set forth in SEQ ID NO: 25, and the light chain variable region comprising the amino acid sequence set forth in SEQ ID NO: 27.

In preferred embodiments, the dissociation constant KD between the anti-human IL6 monoclonal antibody and IL6 is less than 10 nM. In more preferred embodiments, the dissociation constant KD between the anti-human IL6 monoclonal antibody and IL6 is less than 1 nM.

In an embodiment, the anti-human IL6 monoclonal antibody inhibits the promotion of TF-1 cell proliferation by IL6.

In another aspect, the present invention provides an isolated polynucleotide sequence that encodes the anti-human IL6 monoclonal antibody.

In a particular embodiment, the polynucleotide comprises a heavy chain variable region coding sequence that encodes the heavy chain variable region of the anti-human IL6 monoclonal antibody, and a light chain variable region coding sequence that encodes the light chain variable region of the anti-human IL6 monoclonal antibody, wherein the heavy chain variable region coding sequence and the light chain variable region coding sequence are selected from one of the following combinations:
the heavy chain variable region coding sequence comprising the nucleotide sequence set forth in SEQ ID NO: 2, and the light chain variable region coding sequence comprising the nucleotide sequence set forth in SEQ ID NO: 4;
the heavy chain variable region coding sequence comprising the nucleotide sequence set forth in SEQ ID NO: 6, and the light chain variable region coding sequence comprising the nucleotide sequence set forth in SEQ ID NO: 8;
the heavy chain variable region coding sequence comprising the nucleotide sequence set forth in SEQ ID NO: 10, and the light chain variable region coding sequence comprising the nucleotide sequence set forth in SEQ ID NO: 12;
the heavy chain variable region coding sequence comprising the nucleotide sequence set forth in SEQ ID NO: 14, and the light chain variable region coding sequence comprising the nucleotide sequence set forth in SEQ ID NO: 16;
the heavy chain variable region coding sequence comprising the nucleotide sequence set forth in SEQ ID NO: 18, and the light chain variable region coding sequence comprising the nucleotide sequence set forth in SEQ ID NO: 20;
the heavy chain variable region coding sequence comprising the nucleotide sequence set forth in SEQ ID NO: 22, and the light chain variable region coding sequence comprising the nucleotide sequence set forth in SEQ ID NO: 24; and
the heavy chain variable region coding sequence comprising the nucleotide sequence set forth in SEQ ID NO: 26, and the light chain variable region coding sequence comprising the nucleotide sequence set forth in SEQ ID NO: 28.

In another aspect, the present invention provides an expression vector comprising the above polynucleotide.

In another aspect, the present invention provides a host cell comprising the above expression vector.

In preferred embodiments, the host cell is HEK293-6E cell.

In another aspect, the present invention provides a method for preparing an anti-human IL6 monoclonal antibody, comprising transfecting a competent cell with the above expression vector, and culturing the cell.

In another aspect, the present invention provides use of the anti-human IL6 monoclonal antibody, the polynucleotide, the expression vector, the host cell in the preparation of an anti-tumor medicament.

In preferred embodiments, the tumor is selected from multiple myeloma, non-small cell lung cancer, colorectal cancer, renal cancer, prostate cancer, breast cancer and ovarian cancer.

In another aspect, the present invention provides an antitumor pharmaceutical composition, comprising an effective amount of the anti-human IL6 monoclonal antibody, and a pharmaceutically acceptable carrier.

In a further aspect, the present invention provides a method for preparing an anti-human IL6 monoclonal antibody, comprising:
1) immunizing a mouse with human IL6, and generating an immune response against human IL6 in the mouse;
2) fusing the spleen cells obtained from the mouse with myeloma cells, and screening the resulting hybridoma cells, to obtain a positive parent clone that specifically recognizes human IL6;
3) subcloning the positive parent clone, to obtain a stable hybridoma cell line;
4) subjecting the hybridoma cell line to gene sequencing, to obtain the variable region coding sequences of an anti-human IL6 antibody; and
5) using the variable region coding sequences to produce a recombinant antibody, thereby obtaining a functional anti-human IL6 monoclonal antibody.

The anti-IL6 monoclonal antibodies as provided in the present invention have high affinity and high specificity for IL6, and can recognize different epitopes of IL6, respectively, can stimulate or inhibit IL6 downstream pathway, activate or inhibit secretion of cytokines by T cells, for example, inhibit the proliferation of TF-1 cells by inhibiting IL6 downstream pathway. Therefore, the functional monoclonal antibodies against IL6 as provided in the present invention, can inhibit cell proliferation by blocking IL6 signal pathway, and further achieve the purpose of tumor immunotherapy.

### Brief Description of The Drawings

Figure 1 shows the ELISA titer test results of sera from mice immunized with human IL6.
Figure 2 shows the ELISA titer test results of sera from rats immunized with human IL6.
Figure 3 shows the curve of the specific binding of the anti-IL6 monoclonal antibodies of the present invention to IL6 recombinant protein.
Figure 4 shows the curve of the specific inhibition of TF-1 cell proliferation by the anti-IL6 monoclonal antibodies of the present invention.
Figure 5 shows the measurement result of the affinity of the anti-IL6 monoclonal antibodies of the present invention for IL6 protein.

### Detailed Description of the Invention

Unless otherwise specified, the technical and scientific terms used in the present invention have the same meanings as generally understood by those skilled in the art.

As used herein, the term "antibody" generally refers to an immunoglobulin molecule, which is usually a tetramer composed of two identical heavy chains and two identical light chains connected to each other via disulfide bonds. According to the differences in the conservation of the amino acid sequences, heavy chains and light chains are divided into a variable region (V) at the amino terminal and a constant region (C) at the carboxy terminal. In each of the variable regions of heavy chains and light chains, there are three local regions, which have a higher degree of variation in the amino acid composition and arrangement order and are the key positions for the binding of an antibody to an antigen, and therefore are also called complementarity determining regions (CDR). In the context, the three heavy chain complementarity determining regions are called HCDR1, HCDR2 and HCDR3, respectively, and the three light chain complementarity determining regions are called LCDR1, LCDR2 and LCDR3, respectively. The variable regions of one heavy chain and one light chain interact with each other to form an antigen binding site (Fv). Depending on the amino acid sequences of the heavy chain constant regions, antibodies can be divided into different classes. There are five main classes of intact antibodies: IgA, IgD, IgE, IgG and IgM, and some of them can be further divided into subclasses, such as IgG1, IgG2, IgG3, IgG4, IgA and IgA2. For different classes of immunoglobulins, their subunit structures and three-dimensional conformations are known in the art. The present invention is intended to include any of the aforementioned classes or subclasses of antibodies.

As used herein, the term "antibody" is also intended to encompass its digestive fragments or functional variants, for example, antibody fragments capable of binding to IL6 or parts thereof, including, but not limited to Fab (e.g., those obtained by papain digestion of antibodies), F(ab')2 (e.g., those obtained by pepsin digestion), Fv or scFv (e.g., those obtained by molecular biotechnology).

As used herein, the term "monoclonal antibody" refers to an antibody that is homogeneous and is directed only against a certain specific antigenic epitope. In contrast to common polyclonal antibody preparations which typically include different antibodies against different antigenic determinants (epitopes), each monoclonal antibody is directed against a single antigenic determinant on the antigen. The term "monoclonal" refers to the homogenous characteristic of antibodies and should not be explained as antibodies produced by any specific method. The monoclonal antibodies of the present invention are preferably produced by recombinant DNA method, or by screening methods described elsewhere herein.

As used herein, the term "an isolated polynucleotide" refers to a polynucleotide in a state that is not naturally occurring in nature, including polynucleotides isolated from nature (including organisms) by biological techniques, and also including artificially synthesized polynucleotides. An isolated polynucleotide can be genomic DNA, cDNA, mRNA or other synthetic RNA, or a combination thereof. The present invention provides multiple nucleotide sequences encoding heavy chain variable regions and light chain variable regions of anti-human IL6 monoclonal antibodies. It should be noted that those skilled in the art, based on the degeneracy of codons, can design nucleotide sequences that are not completely identical to the nucleotide sequences provided above, but encode the same amino acid sequences, according to the amino acid sequences of heavy chain variable regions and light chain variable regions provided herein. These altered nucleotide sequences are also included in the scope of the present invention.

When relating to polynucleotide, the term "vector" used herein refers to any molecule (e.g., nucleic acids, plasmids, or viruses, etc.) for transferring nucleotide encoding information to a host cell. The term "expression vector" or "expression cassette" refers to a vector suitable for expression of a gene of interest (a nucleotide sequence to be expressed) in a host cell, which generally comprises the elements such as the gene of interest, a promoter, a terminator, and a marker gene.

As used herein, the term "host cell" refers to a cell that has been or can be transformed with a nucleic acid sequence and thereby expresses the selected gene of interest. The term includes the progeny of parent cells, no matter whether the progeny is identical to the original parent cells in morphology or genetic composition, as long as the selected gene of interest is present in the progeny. Commonly used host cells include bacteria, yeasts, mammalian cells, etc.

As used herein, the term "transfection" refers to the uptake of foreign or exogenous DNA by the cell. This technique can be used to introduce one or more exogenous DNA parts into a suitable host cell. Cells can be induced by physicochemical methods (such as by calcium chloride treatment) so that they are in a physiological state most suitable for uptaking and accommodating foreign DNA, i.e. "competent".

When a pharmaceutical composition is mentioned, the term "an effective amount" used herein refers to an amount that can lead to a function or activity in human and/or animal and can be accepted by human and/or animal. The term "a pharmaceutically acceptable carrier" refers to a carrier for administration, including various excipients, diluents, buffers and the like, which are suitable for administration to human and/or animal without excessive adverse side effects, and meanwhile are suitable for maintaining the activity of the drug or active agent contained therein.

Some aspects of the present invention will be described in detail by combining the following examples. Unless otherwise specified, the methods and materials in the Examples described below are commercially available conventional products.

### Examples

### Example 1: Obtainment of human IL6 hybridoma cell line

### 1) Animal immunization

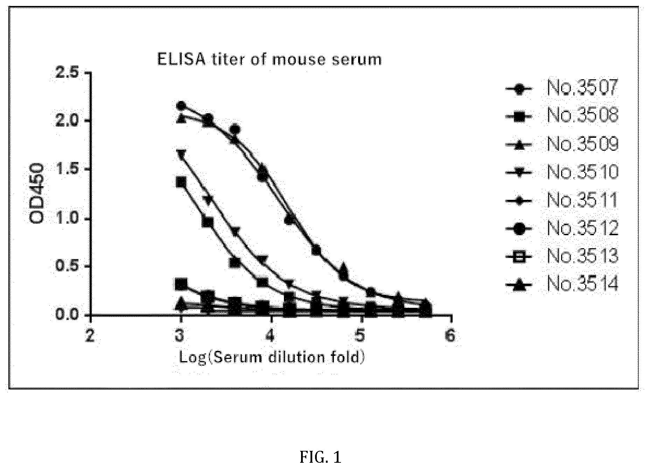

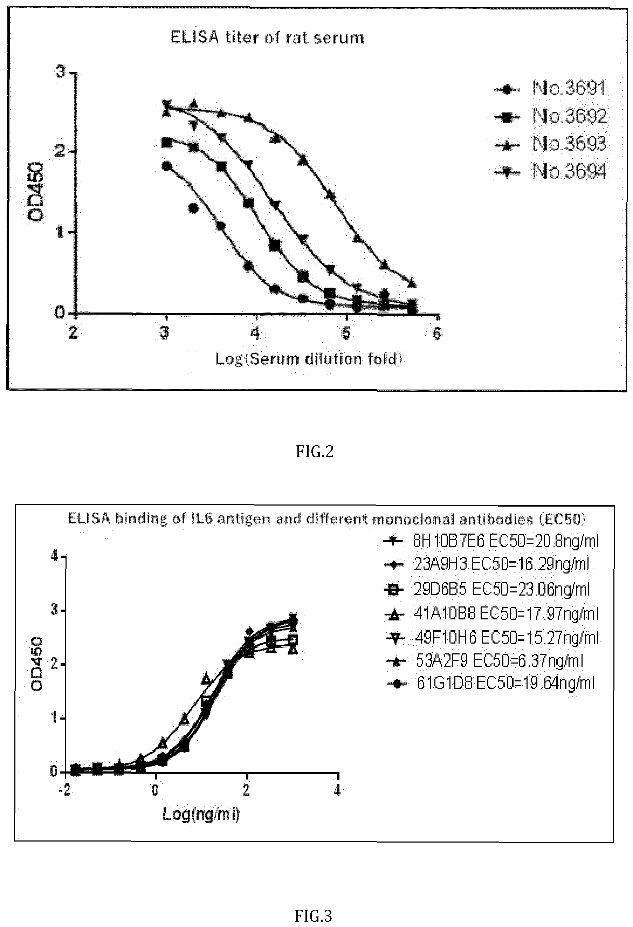

Tag-free human IL6 recombinant protein (GenScript, Cat. No. Z03034) was used as antigen. Female Balb/c and C57bl/6 mice and Wistar rats were subcutaneously immunized with a 1:1 emulsion of 200 µl Freund's complete adjuvant (Sigma-Aldrich) containing 50 µg IL6 protein. Then, a 1:1 emulsion of Freund's incomplete adjuvant (Sigma-Aldrich) containing 25 µg IL6 was injected intraperitoneally/subcutaneously alternately every two weeks up to three times, thereby boosting the mice. Four days before myeloma fusion, a mouse (#3509) and a rat (#3693) showing the highest antibody titer (see Figure 1 and Figure 2, the antibody titer was determined by using serum ELISA method) were boosted by intraperitoneal administration of 25 µg IL6 (without adjuvant).

### 2) Hybridoma fusion and screening

The spleen was extracted and homogenized to produce a single-cell suspension, and meanwhile a single-cell suspension of myeloma cell (SP2/0) was prepared. 8.9×10⁷ spleen cells and 4.1×10⁷ SP2/0 mouse myeloma cells were fused by electrofusion. The fused cells were re-suspended in 100 ml DMEM/10% FBS medium containing hybridoma cell selectors thymidine, hypoxanthine and aminopterin, and a volume of 100 µl was transferred to a 96-well plate with a pipette. The plate was incubated at 37°C in 6% CO₂. After incubation for 7 days, the presence of antibodies against IL6 was detected by using the ELISA binding assay described below.

ELISA binding assay: antibodies in the supernatant were evaluated for the binding ability to IL6 by indirect ELISA. The ELISA plate (Nunc) was coated with 0.5 µg/ml recombinant IL6 protein in PBS at 100 µl/well at 4°C overnight. The plate was washed with PBS-T (0.05% Tween) and blocked with PBST containing 1% BSA at 200 µl/well at 37°C for 0.5 h. The blocking solution was then discarded, and 100 µl hybridoma cell culture supernatant was added to each plate. The incubation was then performed for 1 h at room temperature. The plate was washed with PBST for three times and was incubated with horseradish peroxidase-conjugated goat anti-mouse IgG (Fab-specific) (GenScript) at 100 µl/well at 37°C for 0.5 h. The plate was washed with PBST for five times, and TMB chromogenic solution (GenScript) was then added. Incubation was performed at room temperature in dark for 15 min. The reaction was stopped by adding 50 µl 1M HCl stop solution (Sigma). The plate was read at 450 nm by a Microplate Reader.

### 3) Hybridoma subcloning

Subcloning was performed by limiting dilution method. A hemocytometer was used and cells were subjected to serial dilution in DMEM/10% FBS medium containing hybridoma cell selectors thymine, hypoxanthine and aminopterin to determine the number of cells until the cell density reached 5-15 cells/ml. For each hybridoma, 200 µl cell solution was pipetted into 96 wells at a density of 1-3 cells/well. After culturing the cultures at 37°C in 5% CO₂ for 1 week, the supernatant was subjected to the ELISA binding assay described above to evaluate the presence of antibodies against IL6.

### Example 2: Sequencing of variable regions of monoclonal antibodies and recombinant production of antibodies

After identifying the subtypes of antibodies in the hybridoma cell culture supernatant with a rapid ELISA mouse antibody subtype identification kit (Clonotyping System-HRP, SouthernBiotech), total RNA was extracted from 3×10⁶-5×10⁶ hybridoma cells by using TRIzol (Ambion), and was reverse-transcribed into cDNA by using antibody subtype-specific primers and universal primers (PrimeScript™ 1stStrand cDNA Synthesis Kit, Takara). The mouse immunoglobulin heavy and light chain V-region fragments were then amplified by RACE PCR (GenScript), and the resulting PCR fragments were subcloned into pMD18-T vector system (Takara), and the inserted fragments were sequenced using vector-specific primer pairs. The unique V-region nucleotide/amino acid sequences of the clones 8H10B7E6, 23A9H3, 29D6B5, 41A10B8, 49F10H6, 53A2F9 (rat), and 61G1D8 (rat) were finally obtained. In the amino acid sequences of the heavy chain and light chain, the CDR1 regions are underlined with solid line, the CDR2 regions are underlined with dotted line, and the CDR3 regions are underlined with wavy line.

The amino acid sequence of heavy chain variable region of 8H10B7E6: SEQ ID NO: 1

QVQLQQSGAELAKPGASVKMSCKASGYTLTRYWMHWVKQRPGQGLEWIGYINPITGYTE
NNQKFKDKATLTADKSSSTAYIQLSSLTSEDSAVYFCARGIRGFTYWGHGTLVTVSA

The nucleotide sequence of heavy chain variable region of 8H10B7E6: SEQ ID NO: 2

CAGGTCCAGCTTCAGCAGTCTGGGGCTGAACTGGCAAAACCTGGGGCCTCAGTGAAGATG
TCCTGCAAGGCTTCTGGCTACACCTTAACTAGGTACTGGATGCACTGGGTAAAACAGAGGCCT
GGACAGGGTCTGGAATGGATTGGATACATTAATCCTATCACTGGTTATACTGAGAACAATCA
GAAGTTCAAGGACAAGGCCACATTGACTGCAGACAAGTCCTCCAGCACAGCCTATATACAACT
GAGCAGCCTGACATCTGAGGACTCTGCAGTCTATTTCTGTGCAAGAGGGATACGGGGGTTTAC

TTACTGGGGCCATGGGACTCTGGTCACTGTCTCTGCA

The amino acid sequence of light chain variable region of 8H10B7E6: SEQ ID NO: 3

DIVLTQSPASLAVSLGQRATISCRASENVDNSDNSFMHWYQQKPGQPPKLLIYRASNLDSG
IPARFSGSGSRTDFTLTINPVEADDVATYYCQQTNEAPLTFGAGTKLELK

The nucleotide sequence of light chain variable region of 8H10B7E6: SEQ ID NO: 4

GACATTGTGCTGACCCAATCTCCAGCTTCTTTGGCTGTGTCTCTAGGGCAGAGGGCCACC
ATATCCTGCAGAGCCAGTGAGAATGTTGATAACTCTGACAATAGTTTTATGCACTGGTACCA
ACAGAAACCAGGACAGCCACCCAAACTCCTCATCTATCGTGCATCCAACCTAGATTCTGGGAT
CCCTGCCAGGTTCAGTGGCAGTGGGTCTAGGACAGACTTCACCCTCACCATTAATCCTGTGGA
GGCTGATGATGTTGCAACCTATTACTGTCAGCAAACTAATGAGGCTCCGCTCACGTTCGGTGC
TGGGACCAAGCTGGAGCTGAAA

The amino acid sequence of heavy chain variable region of 23A9H3: SEQ ID NO: 5

QVQLQQSGAELAKPGASVKMSCKTSGYTFTNYWMHWVKQRPGQGLEWIGYVIPSTGYTD
YNQSFKDKATLTADKSSNTAYMQVNSLTSEDSAVYYCASLLPGFAYWGQGTLVTVSA

The nucleotide sequence of heavy chain variable region of 23A9H3: SEQ ID NO: 6

CAGGTCCAGCTTCAGCAGTCTGGGGCTGAACTGGCAAAACCTGGGGCCTCAGTGAAGATG
TCCTGCAAGACTTCTGGCTACACCTTTACTAACTACTGGATGCACTGGGTAAAACAGAGGCCT
GGACAGGGTCTGGAGTGGATTGGATACGTTATTCCTAGCACTGGTTATACTGACTACAATCA
GAGTTTCAAGGACAAGGCCACATTGACTGCAGACAAATCCTCCAACACAGCCTACATGCAAGT
GAACAGCCTGACATCTGAGGACTCTGCAGTCTATTACTGTGCAAGCCTACTGCCAGGTTTTGC
TTACTGGGGCCAAGGGACTCTGGTCACTGTCTCTGCA

The amino acid sequence of light chain variable region of 23A9H3: SEQ ID NO: 7

DVVMTQTPLSLPVSLGDQASISCRSSQSLVDSNGNTYLHWYLQKPGQSPKLLIYKVSNRFS
GVPDRFSGSGSGTDFTLKISRVEAEDLGVYFCSQSTHVPPTFGGGTKLEIK

The nucleotide sequence of light chain variable region of 23A9H3: SEQ ID NO: 8

GATGTTGTGATGACCCAAACTCCACTCTCCCTGCCTGTCAGTCTTGGAGATCAAGCCTCC
ATCTCTTGCAGATCTAGTCAGAGCCTTGTGGACAGTAATGGAAACACTTATTTACATTGGTAC
CTGCAGAAGCCAGGCCAGTCTCCAAAGCTCCTGATCTACAAAGTTTCCAACCGATTTTCTGGG

GTCCCAGACAGGTTCAGTGGCAGTGGATCAGGGACAGATTTCACACTCAAGATCAGCAGAGT
GGAGGCTGAGGATCTGGGGGTTTATTTCTGCTCTCAAAGTACACATGTTCCTCCGACGTTCGG
TGGAGGCACCAAGCTGGAAATCAAA

The amino acid sequence of heavy chain variable region of 29D6B5: SEQ ID NO: 9

QVQLQQSGAELAEPGASVKMSCKASGYNFNNYWMHWVKQGPGQGLEWIGYIDPRTASIY
YNQKFKDKATLTADKSSSTAYMQLSSLTSEDSAVYYCARILYGKYDVWGAGTTVIVSS

The nucleotide sequence of heavy chain variable region of 29D6B5: SEQ ID NO: 10

CAGGTCCAGCTTCAGCAGTCTGGGGCTGAACTGGCAGAACCTGGGGCCTCAGTGAAGATG
TCCTGCAAGGCTTCTGGCTACAACTTTAATAACTATTGGATGCACTGGGTAAAACAGGGGCCT
GGCCAGGGTCTGGAATGGATTGGATACATTGATCCTAGGACTGCTTCTATTTATTACAATCAG
AAGTTCAAAGACAAGGCCACATTGACTGCAGACAAATCCTCCAGCACAGCCTACATGCAACTG
AGCAGCCTGACATCTGAAGACTCTGCAGTCTATTACTGTGCAAGAATCCTCTATGGTAAATAT
GATGTCTGGGGCGCAGGGACCACGGTCATCGTCTCCTCA

The amino acid sequence of light chain variable region of 29D6B5: SEQ ID NO: 11

DVVLTQTPLSLPVSLGNQASISCRSSQSLVDSNGNTYLHWYLQKPGQSPKVLIHKVSNRFS
GVPDRFSGSGSGTDFTLKISRVEAEDLGVYFCSQSTHVPPTFGGGTKLEIK

The nucleotide sequence of light chain variable region of 29D6B5: SEQ ID NO: 12

GATGTTGTGCTGACCCAAACTCCACTCTCCCTGCCTGTCAGTCTTGGAAATCAAGCCTCC
ATCTCTTGCAGATCTAGTCAGAGCCTTGTAGACAGTAATGGAAACACCTATTTACATTGGTAC
CTGCAGAAGCCAGGCCAGTCTCCAAAGGTCCTGATCCACAAAGTTTCCAACCGATTTTCTGGG
GTCCCAGACAGGTTCAGTGGCAGTGGATCAGGGACAGATTTCACACTCAAGATCAGCAGAGT
GGAGGCTGAGGATCTGGGAGTTTATTTCTGCTCTCAAAGTACACATGTTCCTCCGACGTTCGG
TGGAGGCACCAAGCTGGAAATCAAA

The amino acid sequence of heavy chain variable region of 41A10B8: SEQ ID NO: 13

EVKLEESGGGLVQPGGSMKLSCAASGFTFSDAWMDWVRQSPDKGLEWVAEIRSKTYHPA
TYYTKSVRGRFTISRDDSKSSVYLQMNNLGAEDTGIYYCTRPRYYGGYFDYWGQGTTLTVSS

The nucleotide sequence of heavy chain variable region of 41A10B8: SEQ ID NO: 14

GAAGTGAAACTTGAGGAGTCTGGAGGAGGCTTGGTGCAACCTGGAGGATCCATGAAACT

CTCTTGTGCTGCCTCTGGATTCACTTTTAGTGACGCCTGGATGGACTGGGTCCGCCAGTCTCC
AGACAAGGGGCTTGAGTGGGTTGCTGAAATTAGAAGCAAAACTTATCATCCTGCAACATACT
ATACTAAGTCTGTGAGAGGGAGGTTCACCATCTCAAGAGATGATTCCAAAAGTAGTGTCTAC
CTGCAGATGAACAACTTAGGAGCTGAAGACACTGGCATTTATTACTGTACCAGGCCGAGATA
CTACGGGGGTTACTTTGACTACTGGGGCCAAGGCACCACTCTCACAGTCTCCTCA

The amino acid sequence of light chain variable region of 41A10B8: SEQ ID NO: 15

DIVLTQSPASLAVSLGQRATISCRASESVDNYGMSFMNWFQQKPGQPPKLLIYTASNQGSG
VPARFSGSGSGTDFSLNIHPMEEDDTAMYFCQQSKEVPYTFGGGTKLEIK

The nucleotide sequence of light chain variable region of 41A10B8: SEQ ID NO: 16

GACATTGTGCTGACCCAATCTCCAGCTTCTTTGGCTGTGTCTCTAGGGCAGAGGGCCACC
ATCTCCTGCAGAGCCAGCGAAAGTGTTGACAATTATGGCATGAGTTTTATGAACTGGTTCCA
ACAGAAACCAGGACAGCCACCCAAACTCCTCATCTATACTGCTTCCAACCAAGGATCCGGGGT
CCCTGCCAGGTTTAGTGGCAGTGGGTCTGGGACAGACTTCAGCCTCAACATCCATCCTATGGA
GGAGGATGATACTGCAATGTATTTCTGTCAACAAAGTAAGGAGGTCCCGTATACGTTCGGAG
GGGGGACCAAGCTGGAAATAAAA

The amino acid sequence of heavy chain variable region of 49F10H6: SEQ ID NO: 17

QVQLQQPGAELVKPGASVKMSCKASGYTFTNYIIHWIKQTPGQGLEWIGAIYPGNGDTSYS
QKFKDKATLTADRSSSTAYMQLSSLTSEDSAVYYCARGDAGYSAWFAYWGQGTLVTVSA

The nucleotide sequence of heavy chain variable region of 49F10H6: SEQ ID NO: 18

CAGGTGCAACTGCAGCAGCCTGGGGCTGAACTGGTGAAGCCTGGGGCCTCAGTGAAGATG
TCCTGCAAGGCTTCTGGCTACACATTTACCAATTACATTATCCACTGGATAAAACAGACACCT
GGACAGGGCCTGGAATGGATTGGAGCTATTTATCCAGGAAATGGTGATACTTCCTACAGTCA
GAAGTTCAAAGACAAGGCCACATTGACTGCAGACAGATCCTCCAGCACAGCCTACATGCAGCT
CAGCAGCCTGACATCTGAGGACTCTGCGGTCTATTACTGTGCAAGAGGGGATGCGGGTTACTC
CGCCTGGTTTGCTTACTGGGGCCAAGGGACTCTGGTCACTGTCTCTGCA

The amino acid sequence of light chain variable region of 49F10H6: SEQ ID NO: 19

NIVLTQSPASLAVSLGQRATISCSASESVDSYGNNFMHWYQQRPGQPPKLLIYLASKLESGV
PARFSGSGSRTEFTLTIDPVEAEDAATYYCQQNNEDPLTFGAGTKLELK

The nucleotide sequence of light chain variable region of 49F10H6: SEQ ID NO: 20

AACATTGTACTGACCCAATCTCCAGCTTCTTTGGCTGTGTCTCTAGGACAGAGGGCCACC
ATATCCTGCAGTGCCAGTGAAAGTGTTGATAGTTATGGCAATAATTTTATGCACTGGTATCA
GCAGAGACCAGGACAGCCACCCAAACTCCTCATTTATCTTGCATCCAAGCTAGAATCTGGGGT
CCCTGCCAGGTTCAGTGGCAGTGGGTCTAGGACAGAGTTCACCCTCACCATTGACCCTGTGGA
GGCTGAAGATGCTGCAACCTATTACTGTCAGCAAAATAATGAGGATCCGCTCACGTTCGGTGC
TGGGACCAAGCTGGAGCTGAAA

The amino acid sequence of heavy chain variable region of 53A2F9: SEQ ID NO: 21

QVQLKESGPGLVQPSQTLSLTCTVSGFSLTSHTVSWIRQPPGRGLEWMGKMWSNGDTDY
DSAIRSRLSITRDTSKSQVFLKINSLQTEDTAMYFCARYYFSSYGGGYFDYWGQGVMVTVSS

The nucleotide sequence of heavy chain variable region of 53A2F9: SEQ ID NO: 22

CAAGTCCAACTAAAGGAGTCAGGACCTGGTCTGGTACAGCCATCACAGACCCTGTCTCTC
ACCTGCACTGTCTCTGGGTTTTCATTAACCAGCCATACTGTAAGCTGGATTCGGCAGCCTCCA
GGAAGGGGTCTGGAGTGGATGGGAAAAATGTGGAGTAATGGAGACACAGATTATGATTCAGC
TATCAGATCCCGACTGAGCATCACCAGGGACACCTCGAAGAGCCAAGTTTTCTTAAAGATAAA
CAGTCTGCAAACTGAAGACACAGCCATGTACTTCTGTGCCAGATATTACTTTAGCAGCTATGG
AGGTGGCTACTTTGATTACTGGGGCCAAGGAGTCATGGTCACAGTCTCCTCA

The amino acid sequence of light chain variable region of 53A2F9: SEQ ID NO: 23

DTVLTQSPALAVSLGQRVTISCRASKSVSTYMHWYQQKSGQQPKLLIYSASNLESGVPSRF
SGSGSGTDFTLTIDPVEADDIANYYCQQSDELPDTFGAGTKLELK

The nucleotide sequence of light chain variable region of 53A2F9: SEQ ID NO: 24

GACACTGTGCTGACCCAGTCTCCTGCTTTGGCTGTGTCTCTAGGACAGAGGGTCACCATC
TCTTGTAGGGCCAGCAAAAGTGTCAGTACATATATGCACTGGTACCAACAGAAATCGGACA
GCAACCCAAACTCCTGATCTATAGTGCATCCAACCTAGAATCTGGAGTCCCTTCCAGGTTCAG
TGGGAGTGGGTCTGGGACAGACTTTACCCTCACCATAGATCCTGTGGAGGCTGATGACATAGC
AAACTATTACTGTCAGCAGAGTGATGAACTTCCGGACACCTTTGGAGCTGGGACCAAGCTGGA
ACTGAAA

The amino acid sequence of heavy chain variable region of 61G1D8: SEQ ID NO: 25

EVQLLESGGGLVQPGRSMKLSCAASGFTFSSFPMAWVRQTPSKALEWVATISPSGGTSYSR
DSVKGRFTISRDNANSTLCLHMDSLKSEDTATYYCLRERIYNTYFDYWGQGVMVTVSS

The nucleotide sequence of heavy chain variable region of 61G1D8: SEQ ID NO: 26

GAGGTGCAGCTGCTAGAATCTGGGGGCGGTTTAGTGCAGCCTGGAAGGTCCATGAAACTC
TCCTGTGCGGCCTCAGGATTCACTTTCAGTAGCTTTCCAATGGCCTGGGTCCGCCAGACTCCA
TCGAAGGCTCTGGAGTGGGTCGCAACCATTAGTCCTAGTGGTGGTACCTCTTACTCTCGAGAC
TCCGTGAAGGGCCGATTCACTATCTCCAGAGATAATGCAAACAGCACCCTATGTCTACACATG
GACAGTCTGAAGTCTGAGGACACGGCCACTTATTATTGTTTACGAGAAAGGATTTATAACAC
TTACTTTGATTACTGGGGCCAAGGAGTCATGGTCACAGTCTCCTCA

The amino acid sequence of light chain variable region of 61G1D8: SEQ ID NO: 27

DIRMTHSPASLSASLGETVSIECLPSEDISSDLAWYQQKPGKSPQLLIYNANTLPNGVPSRF
SGSRSGAQYFLKINSLQSEDVATYFCQQYDSYPYTFGTGTKLELK

The nucleotide sequence of light chain variable region of 61G1D8: SEQ ID NO: 28

GATATCCGGATGACACACTCTCCAGCTTCCCTGTCTGCATCTCTGGGAGAGACTGTCAGC
ATCGAATGTCTACCAAGTGAGGACATTTCCAGTGATTTAGCATGGTATCAGCAGAAGCCAGG
GAAATCTCCTCAGCTCCTCATCTATAATGCAAATACTTTGCCAAATGGGGTCCCTTCACGGTT
TAGTGGCAGTAGATCTGGCGCACAGTATTTCTAAAAATAAACAGCCTGCAATCTGAGGATG
TCGCGACTTATTTCTGTCAGCAATATGACAGTTATCCGTACACGTTTGGAACTGGGACCAAGC
TGGAACTGAAA

DNA fragment comprising light chain variable region+constant region and DNA fragment comprising heavy chain variable region+constant region were synthesized, respectively, and were inserted into pTT5 expression vectors to form expression plasmids, respectively.

HEK293-6E cells were co-transfected with the expression plasmids above and cultured in a shake flask at 37°C for 10 days. The supernatant was collected for antibody purification. Prior to purification, the tube and protein A column were depyrogenated with 0.2 M NaOH. The column was re-equilibrated with a buffer containing 0.05 M Tris and 1.5 M NaCl (pH 8.0). The harvested cell culture supernatant was then 1: 1 diluted with 2 × the buffer above and sterilized by filtration. The filtered supernatant and the protein A column were incubated at room temperature for 2 h. After washing the column with 1 × the buffer above, IgG was eluted with sterile 0.1 M sodium citrate (pH 3.5). The eluate was collected and neutralized with one-ninth volume of sterile 1M Tris-HCl (pH9). Under sterile conditions, the buffer of the product was exchanged for PBS (pH7.4) to remove any elution buffer and the sample was concentrated. After concentration, antibodies were quantified by OD280nm using an extinction coefficient Ec of 1.43 (0.1%).

Purified antibodies were analyzed by SDS-PAGE using 10% precast gel (GenScript) in BioRad electrophoresis system. The gel was stained with Estin2.0 (GenScript), and the molecular size and purity were estimated by comparing the staining bands with Protein Ladder (GenScript).

### Example 3: Binding of monoclonal antibodies to human IL6 recombinant protein

The purified antibodies were evaluated for the binding ability to IL6 by indirect ELISA. The ELISA plate (Nunc) was coated with 0.5 µg/ml recombinant IL6 protein in PBS at 100 µl/well at 4°C overnight. The plate was washed with PBS-T (0.05% Tween) and was blocked with PBST containing 1% BSA at 200 µl/well at 37°C for 0.5 h. The blocking solution was then discarded. 100 µl 10 µg/ml purified antibody was added to the first well, and 3-fold gradient dilution was performed for 11 test concentration gradients in total. Incubation was then performed at room temperature for 1 h. The plate was washed with PBST for three times and incubated with horseradish peroxidase-conjugated goat anti-mouse/rat IgG (Fab-specific) (GenScript) at 100 µl/well at 37°C for 0.5 h. The plate was washed with PBST for five times, and TMB chromogenic solution (GenScript) was then added and incubation was performed in dark for 15 min at room temperature. The reaction was stopped by adding 50 µl 1M HCl stop solution (Sigma). The plate was read at 450 nm by a microplate reader. The binding ability of the clones 8H10B7E6, 23A9H3, 29D6B5, 41A10B8, 49F10H6, 53A2F9 (rat), and 61G1D8 (rat) for recombinant protein IL6 was shown in Figure 3. EC50, as calculated according to ELISA concentration gradient experiments, showed that these antibodies exhibited relatively high affinity for the antigen (ELISA EC50 was less than 25 ng/ml; especially reached 6.32 ng/ml for 53A2F9, which was equivalent to 0.04 nM).

### Example 4: Identification of the epitopes of monoclonal antibodies

The antigen epitopes of the purified antibodies were evaluated by competitive ELISA. The ELISA plate (Nunc) was coated with 0.5 µg/ml recombinant IL6 protein in PBS at 100 µl/well at 4°C overnight. The plate was washed with PBS-T (0.05% Tween) and was blocked with PBST containing 1% BSA at 200 µl/well at 37°C for 0.5 h. The blocking solution was then discarded. A pair of antibodies to be tested (one of which was biotin-labeled) for competitive experiment were added to each well at 100 µl (10 µg/ml) for each purified antibody. Incubation was then performed at 37°C for 1 h. The plate was washed with PBST for three times and incubated with streptavidin HRP (SA-HRP, GenScript) at 100 µl/well at 37°C for 10 min. The plate was washed with PBST for five times, and TMB chromogenic solution (GenScript) was then added and incubation was performed in dark for 15 min at room temperature. The reaction was stopped by adding 50 µl 1M HCl stop solution (Sigma). The plate was read at 450 nm by a microplate reader. The measurement results show that the clones 8H10B7E6, 23A9H3, 29D6B5 are directed against the same epitope, while 41A10B8, 49F10H6, 53A2F9 (rat), and 61G1D8 (rat) are directed against other four different epitopes, respectively. These results show that the obtained antibodies specific for IL6 have multiple different antigen recognition epitopes. Therefore, the antibodies screened out in the present invention have higher diversity.

### Example 5: Detection of the functions of monoclonal antibodies

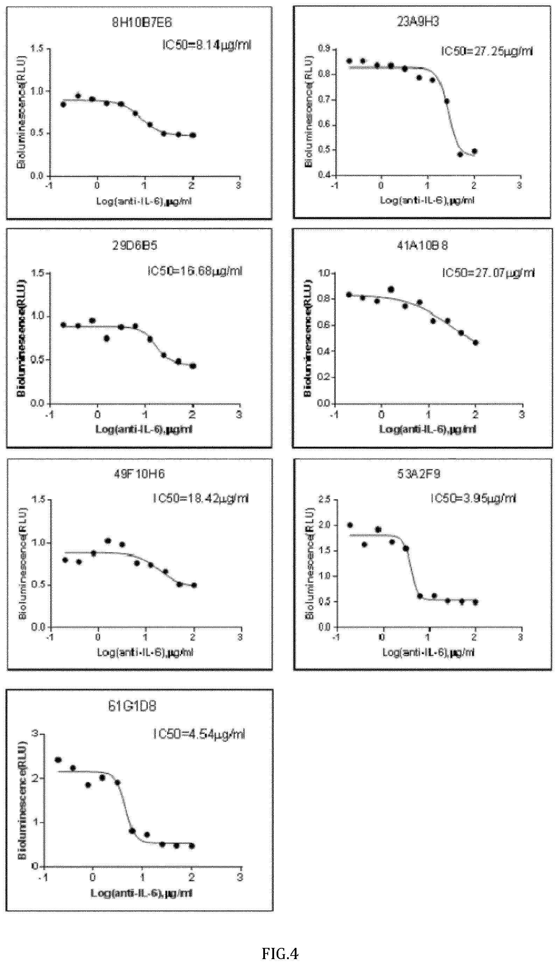

The growth of TF-1 cells is granulocyte macrophage colony-stimulating factor (GM-CSF)-dependent. If this factor is deficient or its function is blocked, the proliferation, differentiation and maturation of the cells will be significantly suppressed. In this monoclonal antibody function experiment, the GM-CSF in the medium was replaced with IL6 protein having the same function, and then anti-IL6 monoclonal antibodies were added to determine whether the monoclonal antibodies could block the biological function of IL6 protein by determining the number of live TF-1 cells after a certain period of culture. TF-1 cells were normally cultured in a medium containing GM-CSF, and the cells were collected prior to the experiment, washed twice with a basal medium without GM-CSF and counted. The cells were plated onto several wells in a 96-well plate at 5,000 cells/well (100 µl). IL6 protein was diluted to 0.4 µg/ml with the basal medium, and was added at 50 µl per well, wherein the protein content per well was 20 ng. The antibody was on another plate, and the dilution was started from 100 µg/ml in a 2-fold manner for 10 gradients. Finally, 50 µl was added to the well. Incubation was performed at 37°C in 5% CO₂ for 7-10 days. 20 µl Promega Substrate Cell Titer 96 Aqueous One Solution Reagent was added to each well. Incubation was performed at 37°C, and the plate was read at 490 nm every half an hour. The best linear data was selected and edited. The inhibitory effect of IL6 antibodies on the growth of TF-1 cells was shown in Figure 4. These results show that the antibodies screened out by the present invention could effectively inhibit cell proliferation, and especially the inhibitory IC50 reached 3.95 µg/ml, and 4.54 µg/ml for the clones 53A2F9 and 61G1D8 antibodies, respectively.

### Example 6: Measurement of affinity of monoclonal antibodies

The chip surface was equilibrated with HBS-EP buffer at a flow rate of 10 µl/min for 5 min, and then the chip was activated by injecting a 1: 1 mixture of "NHS + EDC" at a flow rate of 10 µl/min for 7 min. The capture antibody (Goat anti-mouse IgG) diluted in 10 mM sodium acetate buffer was injected at a flow rate of 10 µl/min for about 7 min for coupling, and finally ethanolamine was injected at a flow rate of 10 µl/min for 7 min for surface blocking.

HBS-EP buffer was used as a sample to perform three pre-cycles to equilibrate the chip so as to make the baseline stable, and the antibodies diluted in HBS-EP buffer was injected at a flow rate of 10 µl/min for 0 to 5 min (the binding signal of the antibody and the antigen was controlled at ∼ 100RU by adjusting the capture time), and the chip was equilibrated with buffer for 1 min. The low-concentration antigen 0.33 nM IL6 was injected at a flow rate of 30 µl/min for 5 min to enable the binding between the antigen and the antibody, and then the buffer was injected at a flow rate of 30 µl/min for 15 min to perform dissociation. 50 mM HCl was injected at a flow rate of 100 µl/min for four times and 10s each time for regeneration, and one cycle was completed. The antigen concentration (such as In IL6) was changed for aother cycle of determination at the next gradient concentration until the determination was completed for all the gradient concentrations (0.33 nM, 1 nM, 3 nM, 9 nM, 27 nM IL6) and repeated concentrations (e.g. 9 nM IL6).

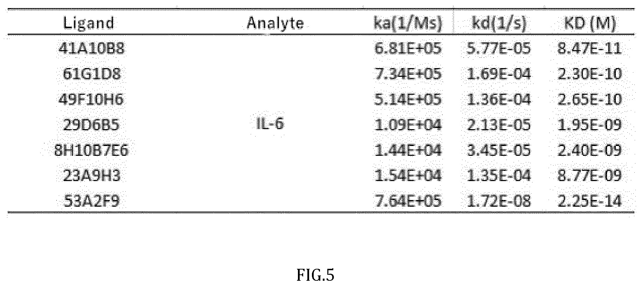

After double deduction of experimental data (control channel and zero concentration), the fitting of a "1: 1 Binding" model was performed in Biacore 8K evaluation software. Biacore 8K was used to determine the affinity of antibodies for IL recombinant protein. As shown in Figure 5, the affinity of monoclonal antibodies specific for IL6 as measured by Biacore reached the magnitude of sub-nM to pM. These results show that the antibodies screened out by the present invention have a very high affinity.

It should be understood by those skilled in the art to which the present invention pertains that the methods and materials described above are merely exemplary and should not be considered as limiting the scope of the present invention.

## Claims

1. An anti-human IL6 monoclonal antibody, comprising a heavy chain variable region and a light chain variable region, wherein the heavy chain variable region comprises HCDR1, HCDR2 and HCDR3, and the light chain variable region comprises LCDR1, LCDR2 and LCDR3, wherein the HCDR1, HCDR2, HCDR3, LCDR1, LCDR2 and LCDR3 are selected from one of the following combinations:
(a) the amino acid sequence of HCDR1 is RYWMH;
the amino acid sequence of HCDR2 is YINPITGYTENNQKFKD;
the amino acid sequence of HCDR3 is GIRGFTY;
the amino acid sequence of LCDR1 is RASENVDNSDNSFMH;
the amino acid sequence of LCDR2 is RASNLDS;
the amino acid sequence of LCDR3 is QQTNEAPLT;
(b) the amino acid sequence of HCDR1 is NYWMH;
the amino acid sequence of HCDR2 is YVIPSTGYTDYNQSFKD;
the amino acid sequence of HCDR3 is LLPGFAY;
the amino acid sequence of LCDR1 is RSSQSLVDSNGNTYLH;
the amino acid sequence of LCDR2 is KVSNRFS;
the amino acid sequence of LCDR3 is SQSTHVPPT;
(c) the amino acid sequence of HCDR1 is NYWMH;
the amino acid sequence of HCDR2 is YIDPRTASIYYNQKFKD;
the amino acid sequence of HCDR3 is ILYGKYDV;
the amino acid sequence of LCDR1 is RSSQSLVDSNGNTYLH;
the amino acid sequence of LCDR2 is KVSNRFS;
the amino acid sequence of LCDR3 is SQSTHVPPT;
(d) the amino acid sequence of HCDR1 is DAWMD;
the amino acid sequence of HCDR2 is EIRSKTYHPATYYTKSVRG;
the amino acid sequence of HCDR3 is PRYYGGYFDY;
the amino acid sequence of LCDR1 is RASESVDNYGMSFMN;
the amino acid sequence of LCDR2 is TASNQGS;
the amino acid sequence of LCDR3 is QQSKEVPYT;
(e) the amino acid sequence of HCDR1 is NYIIH;
the amino acid sequence of HCDR2 is AIYPGNGDTSYSQKFKD;
the amino acid sequence of HCDR3 is GDAGYSAWFAY;
the amino acid sequence of LCDR1 is SASESVDSYGNNFMH;
the amino acid sequence of LCDR2 is LASKLES;
the amino acid sequence of LCDR3 is QQNNEDPLT;
(f) the amino acid sequence of HCDR1 is SHTVS;
the amino acid sequence of HCDR2 is KMWSNGDTDYDSAIRS;
the amino acid sequence of HCDR3 is YYFSSYGGGYFDY;
the amino acid sequence of LCDR1 is RASKSVSTYMH;
the amino acid sequence of LCDR2 is SASNLES;
the amino acid sequence of LCDR3 is QQSDELPDT; and
(g) the amino acid sequence of HCDR1 is SFPMA;
the amino acid sequence of HCDR2 is TISPSGGTSYSRDSVKG;
the amino acid sequence of HCDR3 is ERIYNTYFDY;
the amino acid sequence of LCDR1 is LPSEDISSDLA;
the amino acid sequence of LCDR2 is NANTLPN;
the amino acid sequence of LCDR3 is QQYDSYPYT.

2. The anti-human IL6 monoclonal antibody according to claim 1, wherein the amino acid sequences of its heavy chain variable region and light chain variable region are selected from one of the following combinations:
the heavy chain variable region comprising the amino acid sequence set forth in SEQ ID NO: 1, and the light chain variable region comprising the amino acid sequence set forth in SEQ ID NO: 3;
the heavy chain variable region comprising the amino acid sequence set forth in SEQ ID NO: 5, and the light chain variable region comprising the amino acid sequence set forth in SEQ ID NO: 7;
the heavy chain variable region comprising the amino acid sequence set forth in SEQ ID NO: 9, and the light chain variable region comprising the amino acid sequence set forth in SEQ ID NO: 11;
the heavy chain variable region comprising the amino acid sequence set forth in SEQ ID NO: 13, and the light chain variable region comprising the amino acid sequence set forth in SEQ ID NO: 15;
the heavy chain variable region comprising the amino acid sequence set forth in SEQ ID NO: 17, and the light chain variable region comprising the amino acid sequence set forth in SEQ ID NO: 19;
the heavy chain variable region comprising the amino acid sequence set forth in SEQ ID NO: 21, and the light chain variable region comprising the amino acid sequence set forth in SEQ ID NO: 23; and
the heavy chain variable region comprising the amino acid sequence set forth in SEQ ID NO: 25, and the light chain variable region comprising the amino acid sequence set forth in SEQ ID NO: 27.

3. The anti-human IL6 monoclonal antibody according to claim 1 or 2, wherein the dissociation constant KD between the antibody and IL6 is less than 10 nM.

4. The anti-human IL6 monoclonal antibody according to claim 1 or 2, wherein the dissociation constant KD between the antibody and IL6 is less than 1 nM.

5. The anti-human IL6 monoclonal antibody according to claim 1 or 2, wherein the antibody inhibits the promotion of TF-1 cell proliferation by IL6.

6. An isolated polynucleotide encoding the anti-human IL6 monoclonal antibody according to claim 1 or 2.

7. The polynucleotide according to claim 6, comprising a heavy chain variable region coding sequence that encodes the heavy chain variable region of the anti-human IL6 monoclonal antibody, and a light chain variable region coding sequence that encodes the light chain variable region of the anti-human IL6 monoclonal antibody, wherein the heavy chain variable region coding sequence and the light chain variable region coding sequence are selected from one of the following combinations:
the heavy chain variable region coding sequence comprising the nucleotide sequence set forth in SEQ ID NO: 2, and the light chain variable region coding sequence comprising the nucleotide sequence set forth in SEQ ID NO: 4;
the heavy chain variable region coding sequence comprising the nucleotide sequence set forth in SEQ ID NO: 6, and the light chain variable region coding sequence comprising the nucleotide sequence set forth in SEQ ID NO: 8;
the heavy chain variable region coding sequence comprising the nucleotide sequence set forth in SEQ ID NO: 10, and the light chain variable region coding sequence comprising the nucleotide sequence set forth in SEQ ID NO: 12;
the heavy chain variable region coding sequence comprising the nucleotide sequence set forth in SEQ ID NO: 14, and the light chain variable region coding sequence comprising the nucleotide sequence set forth in SEQ ID NO: 16;
the heavy chain variable region coding sequence comprising the nucleotide sequence set forth in SEQ ID NO: 18, and the light chain variable region coding sequence comprising the nucleotide sequence set forth in SEQ ID NO: 20;
the heavy chain variable region coding sequence comprising the nucleotide sequence set forth in SEQ ID NO: 22, and the light chain variable region coding sequence comprising the nucleotide sequence set forth in SEQ ID NO: 24; and
the heavy chain variable region coding sequence comprising the nucleotide sequence set forth in SEQ ID NO: 26, and the light chain variable region coding sequence comprising the nucleotide sequence set forth in SEQ ID NO: 28.

8. An expression vector comprising the polynucleotide according to claim 6 or 7.

9. A host cell comprising the expression vector according to claim 8.

10. The host cell according to claim 8, which is HEK293-6E cell.

11. A method for preparing an anti-human IL6 monoclonal antibody, comprising transfecting a competent cell with the expression vector according to claim 8, and culturing the cell.

12. Use of the anti-human IL6 monoclonal antibody according to any one of claims 1-5, the polynucleotide according to claim 6 or 7, the expression vector according to claim 8, the host cell according to claim 9 or 10 in the preparation of an anti-tumor medicament.

13. The use according to claim 12, wherein the tumor is selected from multiple myeloma, non-small cell lung cancer, colorectal cancer, renal cancer, prostate cancer, breast cancer and ovarian cancer.

14. An antitumor pharmaceutical composition, comprising an effective amount of the anti-human IL6 monoclonal antibody according to any one of claims 1-5 and a pharmaceutically acceptable carrier.

15. A method for preparing an anti-human IL6 monoclonal antibody, comprising
1) immunizing a mouse with human IL6, and generating an immune response against human IL6 in the mouse;
2) fusing the spleen cells obtained from the mouse with myeloma cells, and screening the resulting hybridoma cells, to obtain a positive parent clone that specifically recognizes human IL6;
3) subcloning the positive parent clone, to obtain a stable hybridoma cell line;
4) subjecting the hybridoma cell line to gene sequencing, to obtain the variable region coding sequences of an anti-human IL6 antibody; and
5) using the variable region coding sequences to produce a recombinant antibody, thereby obtaining a functional anti-human IL6 monoclonal antibody.


SEQUENCE LISTING

<110>  ÄÏ¾©½ðË¹ÈðÉúÎï¿Æ¼¼ÓÐÏÞ¹«Ë¾

<120>  ¿¹ÈËIL6µ¥¿ËÂ¡¿¹Ìå¼°ÆäÖÆ±¸·½·¨°ÍÓÃÍ¾

<130>  P10176-PCT

<150>  CN201711269749.6

<151>  2017-12-05

<160>  28

<170>  PatentIn version 3.5

<210>  1
<211>  116
<212>  PRT
<213>  ÈË¹¤ÐòÁÐ£¨Artificial Sequence£©

<400>  1

Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Thr Leu Thr Arg Tyr
            20                  25                  30

Trp Met His Trp Val Lys Gln Arg Pro Gly Gln Gly Leu Glu Trp Ile
        35                  40                  45

Gly Tyr Ile Asn Pro Ile Thr Gly Tyr Thr Glu Asn Asn Gln Lys Phe
    50                  55                  60

Lys Asp Lys Ala Thr Leu Thr Ala Asp Lys Ser Ser Ser Thr Ala Tyr
65                  70                  75                  80

Ile Gln Leu Ser Ser Leu Thr Ser Glu Asp Ser Ala Val Tyr Phe Cys
                85                  90                  95

Ala Arg Gly Ile Arg Gly Phe Thr Tyr Trp Gly His Gly Thr Leu Val
            100                 105                 110

Thr Val Ser Ala
        115

<210>  2
<211>  348
<212>  DNA
<213>  ÈË¹¤ÐòÁÐ£¨Artificial Sequence£©

<400>  2
caggtccagc ttcagcagtc tggggctgaa ctggcaaaac ctggggcctc agtgaagatg      60

tcctgcaagg cttctggcta caccttaact aggtactgga tgcactgggt aaaacagagg     120

cctggacagg gtctggaatg gattggatac attaatccta tcactggtta tactgagaac     180

aatcagaagt tcaaggacaa ggccacattg actgcagaca agtcctccag cacagcctat     240

atacaactga gcagcctgac atctgaggac tctgcagtct atttctgtgc aagagggata     300

cgggggttta cttactgggg ccatgggact ctggtcactg tctctgca                  348

<210>  3
<211>  111
<212>  PRT
<213>  ÈË¹¤ÐòÁÐ£¨Artificial Sequence£©

<400>  3

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Asn Val Asp Asn Ser
            20                  25                  30

Asp Asn Ser Phe Met His Trp Tyr Gln Gln Lys Pro Gly Gln Pro Pro
        35                  40                  45

Lys Leu Leu Ile Tyr Arg Ala Ser Asn Leu Asp Ser Gly Ile Pro Ala
    50                  55                  60

Arg Phe Ser Gly Ser Gly Ser Arg Thr Asp Phe Thr Leu Thr Ile Asn
65                  70                  75                  80

Pro Val Glu Ala Asp Asp Val Ala Thr Tyr Tyr Cys Gln Gln Thr Asn
                85                  90                  95

Glu Ala Pro Leu Thr Phe Gly Ala Gly Thr Lys Leu Glu Leu Lys
            100                 105                 110

<210>  4
<211>  333
<212>  DNA
<213>  ÈË¹¤ÐòÁÐ£¨Artificial Sequence£©

<400>  4
gacattgtgc tgacccaatc tccagcttct ttggctgtgt ctctagggca gagggccacc      60

atatcctgca gagccagtga gaatgttgat aactctgaca atagttttat gcactggtac     120

caacagaaac caggacagcc acccaaactc ctcatctatc gtgcatccaa cctagattct     180

gggatccctg ccaggttcag tggcagtggg tctaggacag acttcaccct caccattaat     240

cctgtggagg ctgatgatgt tgcaacctat tactgtcagc aaactaatga ggctccgctc 300

acgttcggtg ctgggaccaa gctggagctg aaa 333

<210> 5
<211> 116
<212> PRT
<213> ÈË¹¤ÐòÁÐ£¨Artificial Sequence£©

<400> 5

Gln Val Gln Leu Gln Gln Ser Gly Ala Glu Leu Ala Lys Pro Gly Ala
1               5                   10                  15

Ser Val Lys Met Ser Cys Lys Thr Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Tyr Val Ile Pro Ser Thr Gly Tyr Thr Asp Tyr Asn Gln Ser Phe
    50                  55                  60

Lys Asp Lys Ala Thr Leu Thr Ala Asp Lys Ser Ser Asn Thr Ala Tyr
65                  70                  75                  80

Met Gln Val Asn Ser Leu Thr Ser Glu Asp Ser Ala Val Tyr Tyr Cys
                85                  90                  95

Ala Ser Leu Leu Pro Gly Phe Ala Tyr Trp Gly Gln Gly Thr Leu Val
            100                 105                 110

Thr Val Ser Ala
        115

<210> 6
<211> 348
<212> DNA
<213> ÈË¹¤ÐòÁÐ£¨Artificial Sequence£©

<400> 6
caggtccagc ttcagcagtc tggggctgaa ctggcaaaac ctggggcctc agtgaagatg 60

tcctgcaaga cttctggcta cacctttact aactactgga tgcactgggt aaaacagagg 120

cctggacagg gtctggagtg gattggatac gttattccta gcactggtta tactgactac 180

aatcagagtt tcaaggacaa ggccacattg actgcagaca aatcctccaa cacagcctac 240

atgcaagtga acagcctgac atctgaggac tctgcagtct attactgtgc aagcctactg 300

ccaggttttg cttactgggg ccaagggact ctggtcactg tctctgca 348

<210> 7
<211> 112
<212> PRT
<213> ÈË¹¤ÐòÁÐ£¨Artificial Sequence£©

<400> 7

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val Asp Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Thr Phe Gly Gly Gly Thr Lys Leu Glu Ile Lys
            100                 105                 110

<210> 8
<211> 336
<212> DNA
<213> ÈË¹¤ÐòÁÐ£¨Artificial Sequence£©

<400> 8
gatgttgtga tgacccaaac tccactctcc ctgcctgtca gtcttggaga tcaagcctcc      60

atctcttgca gatctagtca gagccttgtg gacagtaatg gaaacactta tttacattgg     120

tacctgcaga agccaggcca gtctccaaag ctcctgatct acaaagtttc caaccgattt     180

tctggggtcc cagacaggtt cagtggcagt ggatcaggga cagatttcac actcaagatc     240

agcagagtgg aggctgagga tctgggggtt tatttctgct ctcaaagtac acatgttcct     300

ccgacgttcg gtggaggcac caagctggaa atcaaa                               336

<210> 9
<211> 117
<212> PRT
<213> ÈË¹¤ÐòÁÐ£¨Artificial Sequence£©

<400> 9

Gln Val Gln Leu Gln Gln Ser Gly Ala Glu Leu Ala Glu Pro Gly Ala

1               5                   10                  15

Ser Val Lys Met Ser Cys Lys Ala Ser Gly Tyr Asn Phe Asn Asn Tyr
            20                  25                  30

Trp Met His Trp Val Lys Gln Gly Pro Gly Gln Gly Leu Glu Trp Ile
        35                  40                  45

Gly Tyr Ile Asp Pro Arg Thr Ala Ser Ile Tyr Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Leu Tyr Gly Lys Tyr Asp Val Trp Gly Ala Gly Thr Thr
            100                 105                 110

Val Ile Val Ser Ser
        115

<210>  10
<211>  351
<212>  DNA
<213>  ÈË¹¤ÐòÁÐ£¨Artificial Sequence£©

<400>  10
caggtccagc ttcagcagtc tggggctgaa ctggcagaac ctggggcctc agtgaagatg      60

tcctgcaagg cttctggcta caactttaat aactattgga tgcactgggt aaaacagggg     120

cctggccagg gtctggaatg gattggatac attgatccta ggactgcttc tatttattac     180

aatcagaagt tcaaagacaa ggccacattg actgcagaca aatcctccag cacagcctac     240

atgcaactga gcagcctgac atctgaagac tctgcagtct attactgtgc aagaatcctc     300

tatggtaaat atgatgtctg gggcgcaggg accacggtca tcgtctcctc a              351

<210>  11
<211>  112
<212>  PRT
<213>  ÈË¹¤ÐòÁÐ£¨Artificial Sequence£©

<400>  11

Asp Val Val Leu Thr Gln Thr Pro Leu Ser Leu Pro Val Ser Leu Gly
1               5                   10                  15

Asn Gln Ala Ser Ile Ser Cys Arg Ser Ser Gln Ser Leu Val Asp Ser
            20                  25                  30

Asn Gly Asn Thr Tyr Leu His Trp Tyr Leu Gln Lys Pro Gly Gln Ser
        35                  40                  45

Pro Lys Val Leu Ile His Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Thr Phe Gly Gly Gly Thr Lys Leu Glu Ile Lys
            100                 105                 110

<210>  12
<211>  336
<212>  DNA
<213>  ÈË¹¤ÐòÁÐ£¨Artificial Sequence£©

<400>  12
gatgttgtgc tgacccaaac tccactctcc ctgcctgtca gtcttggaaa tcaagcctcc      60

atctcttgca gatctagtca gagccttgta gacagtaatg gaaacaccta tttacattgg     120

tacctgcaga agccaggcca gtctccaaag gtcctgatcc acaaagtttc caaccgattt     180

tctggggtcc cagacaggtt cagtggcagt ggatcaggga cagatttcac actcaagatc     240

agcagagtgg aggctgagga tctgggagtt tatttctgct ctcaaagtac acatgttcct     300

ccgacgttcg gtggaggcac caagctggaa atcaaa                               336

<210>  13
<211>  121
<212>  PRT
<213>  ÈË¹¤ÐòÁÐ£¨Artificial Sequence£©

<400>  13

Glu Val Lys Leu Glu Glu Ser Gly Gly Gly Leu Val Gln Pro Gly Gly
1               5                   10                  15

Ser Met Lys Leu Ser Cys Ala Ala Ser Gly Phe Thr Phe Ser Asp Ala
            20                  25                  30

Trp Met Asp Trp Val Arg Gln Ser Pro Asp Lys Gly Leu Glu Trp Val
        35                  40                  45

Ala Glu Ile Arg Ser Lys Thr Tyr His Pro Ala Thr Tyr Tyr Thr Lys
    50                  55                  60

Ser Val Arg Gly Arg Phe Thr Ile Ser Arg Asp Asp Ser Lys Ser Ser
65                  70                  75                  80

Val Tyr Leu Gln Met Asn Asn Leu Gly Ala Glu Asp Thr Gly Ile Tyr
                85                  90                  95

Tyr Cys Thr Arg Pro Arg Tyr Tyr Gly Gly Tyr Phe Asp Tyr Trp Gly
            100                 105                 110

Gln Gly Thr Thr Leu Thr Val Ser Ser
        115                 120

<210>  14
<211>  363
<212>  DNA
<213>  ÈË¹¤ÐòÁÐ£¨Artificial Sequence£©

<400>  14
gaagtgaaac ttgaggagtc tggaggaggc ttggtgcaac ctggaggatc catgaaactc      60

tcttgtgctg cctctggatt cacttttagt gacgcctgga tggactgggt ccgccagtct     120

ccagacaagg ggcttgagtg ggttgctgaa attagaagca aaacttatca tcctgcaaca     180

tactatacta agtctgtgag agggaggttc accatctcaa gagatgattc caaaagtagt     240

gtctacctgc agatgaacaa cttaggagct gaagacactg gcatttatta ctgtaccagg     300

ccgagatact acgggggtta ctttgactac tggggccaag gcaccactct cacagtctcc     360

tca                                                                   363

<210>  15
<211>  111
<212>  PRT
<213>  ÈË¹¤ÐòÁÐ£¨Artificial Sequence£©

<400>  15

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Asn Tyr
            20                  25                  30

Gly Met Ser Phe Met Asn Trp Phe Gln Gln Lys Pro Gly Gln Pro Pro
        35                  40                  45

Lys Leu Leu Ile Tyr Thr Ala Ser Asn Gln Gly Ser Gly Val Pro Ala
    50                  55                  60

Arg Phe Ser Gly Ser Gly Ser Gly Thr Asp Phe Ser Leu Asn Ile His
65                  70                  75                  80

Pro Met Glu Glu Asp Asp Thr Ala Met Tyr Phe Cys Gln Gln Ser Lys
                85                  90                  95

Glu Val Pro Tyr Thr Phe Gly Gly Gly Thr Lys Leu Glu Ile Lys
            100                 105                 110

<210>  16
<211>  333
<212>  DNA
<213>  ÈË¹¤ÐòÁÐ£¨Artificial Sequence£©

<400>  16
gacattgtgc tgacccaatc tccagcttct ttggctgtgt ctctagggca gagggccacc      60

atctcctgca gagccagcga aagtgttgac aattatggca tgagttttat gaactggttc     120

caacagaaac caggacagcc acccaaactc ctcatctata ctgcttccaa ccaaggatcc     180

ggggtccctg ccaggtttag tggcagtggg tctgggacag acttcagcct caacatccat     240

cctatggagg aggatgatac tgcaatgtat ttctgtcaac aaagtaagga ggtcccgtat     300

acgttcggag gggggaccaa gctggaaata aaa                                  333

<210>  17
<211>  120
<212>  PRT
<213>  ÈË¹¤ÐòÁÐ£¨Artificial Sequence£©

<400>  17

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Asn Tyr
            20                  25                  30

Ile Ile His Trp Ile Lys Gln Thr Pro Gly Gln Gly Leu Glu Trp Ile
        35                  40                  45

Gly Ala Ile Tyr Pro Gly Asn Gly Asp Thr Ser Tyr Ser Gln Lys Phe
    50                  55                  60

Lys Asp Lys Ala Thr Leu Thr Ala Asp Arg Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Asp Ala Gly Tyr Ser Ala Trp Phe Ala Tyr Trp Gly Gln
            100                 105                 110

Gly Thr Leu Val Thr Val Ser Ala
        115                 120

<210>  18
<211>  360
<212>  DNA
<213>  ÈË¹¤ÐòÁÐ£¨Artificial Sequence£©

<400>  18
caggtgcaac tgcagcagcc tggggctgaa ctggtgaagc ctggggcctc agtgaagatg        60

tcctgcaagg cttctggcta cacatttacc aattacatta tccactggat aaaacagaca       120

cctggacagg gcctggaatg gattggagct atttatccag gaaatggtga tacttcctac       180

agtcagaagt tcaaagacaa ggccacattg actgcagaca gatcctccag cacagcctac       240

atgcagctca gcagcctgac atctgaggac tctgcggtct attactgtgc aagaggggat       300

gcgggttact ccgcctggtt tgcttactgg ggccaaggga ctctggtcac tgtctctgca       360

<210>  19
<211>  111
<212>  PRT
<213>  ÈË¹¤ÐòÁÐ£¨Artificial Sequence£©

<400>  19

Asn Ile Val Leu Thr Gln Ser Pro Ala Ser Leu Ala Val Ser Leu Gly
1               5                   10                  15

Gln Arg Ala Thr Ile Ser Cys Ser Ala Ser Glu Ser Val Asp Ser Tyr
            20                  25                  30

Gly Asn Asn Phe Met His Trp Tyr Gln Gln Arg Pro Gly Gln Pro Pro
        35                  40                  45

Lys Leu Leu Ile Tyr Leu Ala Ser Lys Leu Glu Ser Gly Val Pro Ala
    50                  55                  60

Arg Phe Ser Gly Ser Gly Ser Arg Thr Glu Phe Thr Leu Thr Ile Asp
65                  70                  75                  80

Pro Val Glu Ala Glu Asp Ala Ala Thr Tyr Tyr Cys Gln Gln Asn Asn
                85                  90                  95

Glu Asp Pro Leu Thr Phe Gly Ala Gly Thr Lys Leu Glu Leu Lys
            100                 105                 110

<210>  20
<211>  333
<212>  DNA
<213>  ÈË¹¤ÐòÁÐ£¨Artificial Sequence£©

<400>  20
aacattgtac tgacccaatc tccagcttct ttggctgtgt ctctaggaca gagggccacc      60

atatcctgca gtgccagtga aagtgttgat agttatggca ataattttat gcactggtat     120

cagcagagac caggacagcc acccaaactc ctcatttatc ttgcatccaa gctagaatct     180

ggggtccctg ccaggttcag tggcagtggg tctaggacag agttcaccct caccattgac     240

cctgtggagg ctgaagatgc tgcaacctat tactgtcagc aaaataatga ggatccgctc     300

acgttcggtg ctgggaccaa gctggagctg aaa                                  333


<210>  21
<211>  121
<212>  PRT
<213>  ÈË¹¤ÐòÁÐ£¨Artificial Sequence£©

<400>  21

Gln Val Gln Leu Lys Glu Ser Gly Pro Gly Leu Val Gln Pro Ser Gln
1               5                   10                  15


Thr Leu Ser Leu Thr Cys Thr Val Ser Gly Phe Ser Leu Thr Ser His
            20                  25                  30


Thr Val Ser Trp Ile Arg Gln Pro Pro Gly Arg Gly Leu Glu Trp Met
        35                  40                  45


Gly Lys Met Trp Ser Asn Gly Asp Thr Asp Tyr Asp Ser Ala Ile Arg
    50                  55                  60


Ser Arg Leu Ser Ile Thr Arg Asp Thr Ser Lys Ser Gln Val Phe Leu
65                  70                  75                  80


Lys Ile Asn Ser Leu Gln Thr Glu Asp Thr Ala Met Tyr Phe Cys Ala
                85                  90                  95


Arg Tyr Tyr Phe Ser Ser Tyr Gly Gly Gly Tyr Phe Asp Tyr Trp Gly
            100                 105                 110


Gln Gly Val Met Val Thr Val Ser Ser
        115                 120


<210>  22
<211>  363
<212>  DNA
<213>  ÈË¹¤ÐòÁÐ£¨Artificial Sequence£©

<400>  22
caagtccaac taaaggagtc aggacctggt ctggtacagc catcacagac cctgtctctc      60

acctgcactg tctctgggtt ttcattaacc agccatactg taagctggat tcggcagcct     120

ccaggaaggg gtctggagtg gatgggaaaa atgtggagta atggagacac agattatgat 180

tcagctatca gatcccgact gagcatcacc agggacacct cgaagagcca agttttctta 240

aagataaaca gtctgcaaac tgaagacaca gccatgtact tctgtgccag atattacttt 300

agcagctatg gaggtggcta ctttgattac tggggccaag gagtcatggt cacagtctcc 360

tca 363

<210> 23
<211> 106
<212> PRT
<213> ÈË¹¤ÐòÁÐ£¨Artificial Sequence£©

<400> 23

Asp Thr Val Leu Thr Gln Ser Pro Ala Leu Ala Val Ser Leu Gly Gln
1               5                   10                  15

Arg Val Thr Ile Ser Cys Arg Ala Ser Lys Ser Val Ser Thr Tyr Met
            20                  25                  30

His Trp Tyr Gln Gln Lys Ser Gly Gln Gln Pro Lys Leu Leu Ile Tyr
        35                  40                  45

Ser Ala Ser Asn Leu Glu Ser Gly Val Pro Ser Arg Phe Ser Gly Ser
    50                  55                  60

Gly Ser Gly Thr Asp Phe Thr Leu Thr Ile Asp Pro Val Glu Ala Asp
65                  70                  75                  80

Asp Ile Ala Asn Tyr Tyr Cys Gln Gln Ser Asp Glu Leu Pro Asp Thr
                85                  90                  95

Phe Gly Ala Gly Thr Lys Leu Glu Leu Lys
            100                 105

<210> 24
<211> 318
<212> DNA
<213> ÈË¹¤ÐòÁÐ£¨Artificial Sequence£©

<400> 24
gacactgtgc tgacccagtc tcctgctttg gctgtgtctc taggacagag ggtcaccatc 60

tcttgtaggg ccagcaaaag tgtcagtaca tatatgcact ggtaccaaca gaaatcggga 120

cagcaaccca aactcctgat ctatagtgca tccaacctag aatctggagt cccttccagg 180

ttcagtggga gtgggtctgg gacagacttt accctcacca tagatcctgt ggaggctgat 240

gacatagcaa actattactg tcagcagagt gatgaacttc cggacacctt tggagctggg 300

accaagctgg aactgaaa                                                    318

<210>  25
<211>  119
<212>  PRT
<213>  ÈË¹¤ÐòÁÐ£¨Artificial Sequence£©

<400>  25

Glu Val Gln Leu Leu Glu Ser Gly Gly Gly Leu Val Gln Pro Gly Arg
1               5                   10                  15

Ser Met Lys Leu Ser Cys Ala Ala Ser Gly Phe Thr Phe Ser Ser Phe
            20                  25                  30

Pro Met Ala Trp Val Arg Gln Thr Pro Ser Lys Ala Leu Glu Trp Val
        35                  40                  45

Ala Thr Ile Ser Pro Ser Gly Gly Thr Ser Tyr Ser Arg Asp Ser Val
    50                  55                  60

Lys Gly Arg Phe Thr Ile Ser Arg Asp Asn Ala Asn Ser Thr Leu Cys
65                  70                  75                  80

Leu His Met Asp Ser Leu Lys Ser Glu Asp Thr Ala Thr Tyr Tyr Cys
                85                  90                  95

Leu Arg Glu Arg Ile Tyr Asn Thr Tyr Phe Asp Tyr Trp Gly Gln Gly
            100                 105                 110

Val Met Val Thr Val Ser Ser
        115

<210>  26
<211>  357
<212>  DNA
<213>  ÈË¹¤ÐòÁÐ£¨Artificial Sequence£©

<400>  26
gaggtgcagc tgctagaatc tgggggcggt ttagtgcagc ctggaaggtc catgaaactc      60

tcctgtgcgg cctcaggatt cactttcagt agctttccaa tggcctgggt ccgccagact     120

ccatcgaagg ctctggagtg ggtcgcaacc attagtccta gtggtggtac ctcttactct     180

cgagactccg tgaagggccg attcactatc tccagagata atgcaaacag caccctatgt     240

ctacacatgg acagtctgaa gtctgaggac acggccactt attattgttt acgagaaagg     300

atttataaca cttactttga ttactggggc caaggagtca tggtcacagt ctcctca        357

<210>  27
<211>  107

<212>  PRT
<213>  ÈË¹¤ÐòÁÐ£¨Artificial Sequence£©

<400>  27

Asp Ile Arg Met Thr His Ser Pro Ala Ser Leu Ser Ala Ser Leu Gly
1               5                   10                  15

Glu Thr Val Ser Ile Glu Cys Leu Pro Ser Glu Asp Ile Ser Ser Asp
            20                  25                  30

Leu Ala Trp Tyr Gln Gln Lys Pro Gly Lys Ser Pro Gln Leu Leu Ile
        35                  40                  45

Tyr Asn Ala Asn Thr Leu Pro Asn Gly Val Pro Ser Arg Phe Ser Gly
    50                  55                  60

Ser Arg Ser Gly Ala Gln Tyr Phe Leu Lys Ile Asn Ser Leu Gln Ser
65                  70                  75                  80

Glu Asp Val Ala Thr Tyr Phe Cys Gln Gln Tyr Asp Ser Tyr Pro Tyr
                85                  90                  95

Thr Phe Gly Thr Gly Thr Lys Leu Glu Leu Lys
            100                 105


<210>  28
<211>  321
<212>  DNA
<213>  ÈË¹¤ÐòÁÐ£¨Artificial Sequence£©

<400>  28
gatatccgga tgacacactc tccagcttcc ctgtctgcat ctctgggaga gactgtcagc      60

atcgaatgtc taccaagtga ggacatttcc agtgatttag catggtatca gcagaagcca     120

gggaaatctc ctcagctcct catctataat gcaaatactt tgccaaatgg ggtcccttca     180

cggtttagtg gcagtagatc tggcgcacag tattttctaa aaataaacag cctgcaatct     240

gaggatgtcg cgacttattt ctgtcagcaa tatgacagtt atccgtacac gtttggaact     300

gggaccaagc tggaactgaa a                                               321

SEQUENCE LISTING

<110>  Nanjingjinsirui Science & Technology Biology Corp.

<120>  ANTI-HUMAN IL6 MONOCLONAL ANTIBODIES, PREPARATION METHOD THEREFOR AND
USE THEREOF

<130>  NNJ20P01EP1

<150>  18886699.0

<151>  2018-12-05

<160>  66

<170>  PatentIn version 3.5

<210>  1
<211>  116
<212>  PRT
<213>  Artificial Sequence

<400>  1

Gln Val Gln Leu Gln Gln Ser Gly Ala Glu Leu Ala Lys Pro Gly Ala
1               5                   10                  15

Ser Val Lys Met Ser Cys Lys Ala Ser Gly Tyr Thr Leu Thr Arg Tyr
            20                  25                  30

Trp Met His Trp Val Lys Gln Arg Pro Gly Gln Gly Leu Glu Trp Ile
        35                  40                  45

Gly Tyr Ile Asn Pro Ile Thr Gly Tyr Thr Glu Asn Asn Gln Lys Phe
    50                  55                  60

Lys Asp Lys Ala Thr Leu Thr Ala Asp Lys Ser Ser Ser Thr Ala Tyr
65                  70                  75                  80

Ile Gln Leu Ser Ser Leu Thr Ser Glu Asp Ser Ala Val Tyr Phe Cys
                85                  90                  95

Ala Arg Gly Ile Arg Gly Phe Thr Tyr Trp Gly His Gly Thr Leu Val
            100                 105                 110

Thr Val Ser Ala
        115

<210>  2
<211>  348
<212>  DNA

<213>  Artificial Sequence

<400>  2
caggtccagc ttcagcagtc tggggctgaa ctggcaaaac ctggggcctc agtgaagatg      60

tcctgcaagg cttctggcta caccttaact aggtactgga tgcactgggt aaaacagagg     120

cctggacagg gtctggaatg gattggatac attaatccta tcactggtta tactgagaac     180

aatcagaagt tcaaggacaa ggccacattg actgcagaca agtcctccag cacagcctat     240

atacaactga gcagcctgac atctgaggac tctgcagtct atttctgtgc aagagggata     300

cgggggttta cttactgggg ccatgggact ctggtcactg tctctgca                  348


<210>  3
<211>  111
<212>  PRT
<213>  Artificial Sequence

<400>  3

Asp Ile Val Leu Thr Gln Ser Pro Ala Ser Leu Ala Val Ser Leu Gly
1               5                   10                  15


Gln Arg Ala Thr Ile Ser Cys Arg Ala Ser Glu Asn Val Asp Asn Ser
            20                  25                  30


Asp Asn Ser Phe Met His Trp Tyr Gln Gln Lys Pro Gly Gln Pro Pro
        35                  40                  45


Lys Leu Leu Ile Tyr Arg Ala Ser Asn Leu Asp Ser Gly Ile Pro Ala
    50                  55                  60


Arg Phe Ser Gly Ser Gly Ser Arg Thr Asp Phe Thr Leu Thr Ile Asn
65                  70                  75                  80


Pro Val Glu Ala Asp Asp Val Ala Thr Tyr Tyr Cys Gln Gln Thr Asn
                85                  90                  95


Glu Ala Pro Leu Thr Phe Gly Ala Gly Thr Lys Leu Glu Leu Lys
            100                 105                 110


<210>  4
<211>  333
<212>  DNA
<213>  Artificial Sequence

<400>  4
gacattgtgc tgacccaatc tccagcttct ttggctgtgt ctctagggca gagggccacc      60

atatcctgca gagccagtga gaatgttgat aactctgaca atagttttat gcactggtac     120

caacagaaac caggacagcc acccaaactc ctcatctatc gtgcatccaa cctagattct     180

gggatccctg ccaggttcag tggcagtggg tctaggacag acttcaccct caccattaat 240

cctgtggagg ctgatgatgt tgcaacctat tactgtcagc aaactaatga ggctccgctc 300

acgttcggtg ctgggaccaa gctggagctg aaa 333

<210> 5
<211> 116
<212> PRT
<213> Artificial Sequence

<400> 5

Gln Val Gln Leu Gln Gln Ser Gly Ala Glu Leu Ala Lys Pro Gly Ala
1               5                   10                  15

Ser Val Lys Met Ser Cys Lys Thr Ser Gly Tyr Thr Phe Thr Asn Tyr
            20                  25                  30

Trp Met His Trp Val Lys Gln Arg Pro Gly Gln Gly Leu Glu Trp Ile
        35                  40                  45

Gly Tyr Val Ile Pro Ser Thr Gly Tyr Thr Asp Tyr Asn Gln Ser Phe
    50                  55                  60

Lys Asp Lys Ala Thr Leu Thr Ala Asp Lys Ser Ser Asn Thr Ala Tyr
65                  70                  75                  80

Met Gln Val Asn Ser Leu Thr Ser Glu Asp Ser Ala Val Tyr Tyr Cys
                85                  90                  95

Ala Ser Leu Leu Pro Gly Phe Ala Tyr Trp Gly Gln Gly Thr Leu Val
            100                 105                 110

Thr Val Ser Ala
        115

<210> 6
<211> 348
<212> DNA
<213> Artificial Sequence

<400> 6
caggtccagc ttcagcagtc tggggctgaa ctggcaaaac ctggggcctc agtgaagatg 60

tcctgcaaga cttctggcta cacctttact aactactgga tgcactgggt aaaacagagg 120

cctggacagg gtctggagtg gattggatac gttattccta gcactggtta tactgactac 180

aatcagagtt tcaaggacaa ggccacattg actgcagaca aatcctccaa cacagcctac 240

atgcaagtga acagcctgac atctgaggac tctgcagtct attactgtgc aagcctactg 300

ccaggttttg cttactgggg ccaagggact ctggtcactg tctctgca 348

<210>  7
<211>  112
<212>  PRT
<213>  Artificial Sequence

<400>  7

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val Asp Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Thr Phe Gly Gly Gly Thr Lys Leu Glu Ile Lys
            100                 105                 110

<210>  8
<211>  336
<212>  DNA
<213>  Artificial Sequence

<400>  8
gatgttgtga tgacccaaac tccactctcc ctgcctgtca gtcttggaga tcaagcctcc      60

atctcttgca gatctagtca gagccttgtg gacagtaatg gaaacactta tttacattgg     120

tacctgcaga agccaggcca gtctccaaag ctcctgatct acaaagtttc caaccgattt     180

tctggggtcc cagacaggtt cagtggcagt ggatcaggga cagatttcac actcaagatc     240

agcagagtgg aggctgagga tctgggggtt tatttctgct ctcaaagtac acatgttcct     300

ccgacgttcg gtggaggcac caagctggaa atcaaa                               336

<210>  9
<211>  117
<212>  PRT
<213>  Artificial Sequence

<400>  9

Gln Val Gln Leu Gln Gln Ser Gly Ala Glu Leu Ala Glu Pro Gly Ala
1               5                   10                  15

Ser Val Lys Met Ser Cys Lys Ala Ser Gly Tyr Asn Phe Asn Asn Tyr
            20                  25                  30

Trp Met His Trp Val Lys Gln Gly Pro Gly Gln Gly Leu Glu Trp Ile
        35                  40                  45

Gly Tyr Ile Asp Pro Arg Thr Ala Ser Ile Tyr Tyr Asn Gln Lys Phe
    50                  55                  60

Lys Asp Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ile Leu Tyr Gly Lys Tyr Asp Val Trp Gly Ala Gly Thr Thr
            100                 105                 110

Val Ile Val Ser Ser
        115

<210>  10
<211>  351
<212>  DNA
<213>  Artificial Sequence

<400>  10
caggtccagc ttcagcagtc tggggctgaa ctggcagaac ctggggcctc agtgaagatg      60

tcctgcaagg cttctggcta caactttaat aactattgga tgcactgggt aaaacagggg     120

cctggccagg gtctggaatg gattggatac attgatccta ggactgcttc tatttattac     180

aatcagaagt tcaaagacaa ggccacattg actgcagaca aatcctccag cacagcctac     240

atgcaactga gcagcctgac atctgaagac tctgcagtct attactgtgc aagaatcctc     300

tatggtaaat atgatgtctg gggcgcaggg accacggtca tcgtctcctc a              351

<210>  11
<211>  112
<212>  PRT
<213>  Artificial Sequence

<400>  11

Asp Val Val Leu Thr Gln Thr Pro Leu Ser Leu Pro Val Ser Leu Gly
1               5                   10                  15

Asn Gln Ala Ser Ile Ser Cys Arg Ser Ser Gln Ser Leu Val Asp Ser

20                  25                  30

Asn Gly Asn Thr Tyr Leu His Trp Tyr Leu Gln Lys Pro Gly Gln Ser
        35                  40                  45

Pro Lys Val Leu Ile His Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Ser
                85                  90                  95

Thr His Val Pro Pro Thr Phe Gly Gly Gly Thr Lys Leu Glu Ile Lys
            100                 105                 110

<210>  12
<211>  336
<212>  DNA
<213>  Artificial Sequence

<400>  12
gatgttgtgc tgacccaaac tccactctcc ctgcctgtca gtcttggaaa tcaagcctcc      60

atctcttgca gatctagtca gagccttgta gacagtaatg gaaacaccta tttacattgg     120

tacctgcaga agccaggcca gtctccaaag gtcctgatcc acaaagtttc caaccgattt     180

tctggggtcc cagacaggtt cagtggcagt ggatcaggga cagatttcac actcaagatc     240

agcagagtgg aggctgagga tctgggagtt tatttctgct ctcaaagtac acatgttcct     300

ccgacgttcg gtggaggcac caagctggaa atcaaa                               336

<210>  13
<211>  121
<212>  PRT
<213>  Artificial Sequence

<400>  13

Glu Val Lys Leu Glu Glu Ser Gly Gly Gly Leu Val Gln Pro Gly Gly
1               5                   10                  15

Ser Met Lys Leu Ser Cys Ala Ala Ser Gly Phe Thr Phe Ser Asp Ala
            20                  25                  30

Trp Met Asp Trp Val Arg Gln Ser Pro Asp Lys Gly Leu Glu Trp Val
        35                  40                  45

Ala Glu Ile Arg Ser Lys Thr Tyr His Pro Ala Thr Tyr Tyr Thr Lys
    50                  55                  60

Ser Val Arg Gly Arg Phe Thr Ile Ser Arg Asp Asp Ser Lys Ser Ser
65                  70                  75                  80

Val Tyr Leu Gln Met Asn Asn Leu Gly Ala Glu Asp Thr Gly Ile Tyr
                85                  90                  95

Tyr Cys Thr Arg Pro Arg Tyr Tyr Gly Gly Tyr Phe Asp Tyr Trp Gly
            100                 105                 110

Gln Gly Thr Thr Leu Thr Val Ser Ser
        115                 120

<210>  14
<211>  363
<212>  DNA
<213>  Artificial Sequence

<400>  14
gaagtgaaac ttgaggagtc tggaggaggc ttggtgcaac ctggaggatc catgaaactc      60

tcttgtgctg cctctggatt cacttttagt gacgcctgga tggactgggt ccgccagtct     120

ccagacaagg ggcttgagtg ggttgctgaa attagaagca aaacttatca tcctgcaaca     180

tactatacta agtctgtgag agggaggttc accatctcaa gagatgattc caaaagtagt     240

gtctacctgc agatgaacaa cttaggagct gaagacactg gcatttatta ctgtaccagg     300

ccgagatact acgggggtta ctttgactac tggggccaag gcaccactct cacagtctcc     360

tca                                                                   363

<210>  15
<211>  111
<212>  PRT
<213>  Artificial Sequence

<400>  15

Asp Ile Val Leu Thr Gln Ser Pro Ala Ser Leu Ala Val Ser Leu Gly
1               5                   10                  15

Gln Arg Ala Thr Ile Ser Cys Arg Ala Ser Glu Ser Val Asp Asn Tyr
            20                  25                  30

Gly Met Ser Phe Met Asn Trp Phe Gln Gln Lys Pro Gly Gln Pro Pro
        35                  40                  45

Lys Leu Leu Ile Tyr Thr Ala Ser Asn Gln Gly Ser Gly Val Pro Ala
    50                  55                  60

Arg Phe Ser Gly Ser Gly Ser Gly Thr Asp Phe Ser Leu Asn Ile His

65                  70                  75                  80

Pro Met Glu Glu Asp Asp Thr Ala Met Tyr Phe Cys Gln Gln Ser Lys
                85                  90                  95

Glu Val Pro Tyr Thr Phe Gly Gly Gly Thr Lys Leu Glu Ile Lys
            100                 105                 110

<210>  16
<211>  333
<212>  DNA
<213>  Artificial Sequence

<400>  16
gacattgtgc tgacccaatc tccagcttct ttggctgtgt ctctagggca gagggccacc      60

atctcctgca gagccagcga aagtgttgac aattatggca tgagttttat gaactggttc     120

caacagaaac caggacagcc acccaaactc ctcatctata ctgcttccaa ccaaggatcc     180

ggggtccctg ccaggtttag tggcagtggg tctgggacag acttcagcct caacatccat     240

cctatggagg aggatgatac tgcaatgtat ttctgtcaac aaagtaagga ggtcccgtat     300

acgttcggag gggggaccaa gctggaaata aaa                                  333

<210>  17
<211>  120
<212>  PRT
<213>  Artificial Sequence

<400>  17

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Asn Tyr
            20                  25                  30

Ile Ile His Trp Ile Lys Gln Thr Pro Gly Gln Gly Leu Glu Trp Ile
        35                  40                  45

Gly Ala Ile Tyr Pro Gly Asn Gly Asp Thr Ser Tyr Ser Gln Lys Phe
    50                  55                  60

Lys Asp Lys Ala Thr Leu Thr Ala Asp Arg Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Gly Asp Ala Gly Tyr Ser Ala Trp Phe Ala Tyr Trp Gly Gln
            100                 105                 110

Gly Thr Leu Val Thr Val Ser Ala
        115                 120

<210>  18
<211>  360
<212>  DNA
<213>  Artificial Sequence

<400>  18
caggtgcaac tgcagcagcc tggggctgaa ctggtgaagc ctggggcctc agtgaagatg        60

tcctgcaagg cttctggcta cacatttacc aattacatta tccactggat aaaacagaca       120

cctggacagg gcctggaatg gattggagct atttatccag gaaatggtga tacttcctac       180

agtcagaagt tcaaagacaa ggccacattg actgcagaca gatcctccag cacagcctac       240

atgcagctca gcagcctgac atctgaggac tctgcggtct attactgtgc aagaggggat       300

gcgggttact ccgcctggtt tgcttactgg ggccaaggga ctctggtcac tgtctctgca       360

<210>  19
<211>  111
<212>  PRT
<213>  Artificial Sequence

<400>  19

Asn Ile Val Leu Thr Gln Ser Pro Ala Ser Leu Ala Val Ser Leu Gly
1               5                   10                  15

Gln Arg Ala Thr Ile Ser Cys Ser Ala Ser Glu Ser Val Asp Ser Tyr
            20                  25                  30

Gly Asn Asn Phe Met His Trp Tyr Gln Gln Arg Pro Gly Gln Pro Pro
        35                  40                  45

Lys Leu Leu Ile Tyr Leu Ala Ser Lys Leu Glu Ser Gly Val Pro Ala
    50                  55                  60

Arg Phe Ser Gly Ser Gly Ser Arg Thr Glu Phe Thr Leu Thr Ile Asp
65                  70                  75                  80

Pro Val Glu Ala Glu Asp Ala Ala Thr Tyr Tyr Cys Gln Gln Asn Asn
                85                  90                  95

Glu Asp Pro Leu Thr Phe Gly Ala Gly Thr Lys Leu Glu Leu Lys
            100                 105                 110

<210>  20
<211>  333
<212>  DNA

<213>  Artificial Sequence

<400>  20
aacattgtac tgacccaatc tccagcttct ttggctgtgt ctctaggaca gagggccacc      60

atatcctgca gtgccagtga aagtgttgat agttatggca ataattttat gcactggtat     120

cagcagagac caggacagcc acccaaactc ctcatttatc ttgcatccaa gctagaatct     180

ggggtccctg ccaggttcag tggcagtggg tctaggacag agttcaccct caccattgac     240

cctgtggagg ctgaagatgc tgcaacctat tactgtcagc aaaataatga ggatccgctc     300

acgttcggtg ctgggaccaa gctggagctg aaa                                  333


<210>  21
<211>  121
<212>  PRT
<213>  Artificial Sequence

<400>  21

Gln Val Gln Leu Lys Glu Ser Gly Pro Gly Leu Val Gln Pro Ser Gln
1               5                   10                  15


Thr Leu Ser Leu Thr Cys Thr Val Ser Gly Phe Ser Leu Thr Ser His
            20                  25                  30


Thr Val Ser Trp Ile Arg Gln Pro Pro Gly Arg Gly Leu Glu Trp Met
        35                  40                  45


Gly Lys Met Trp Ser Asn Gly Asp Thr Asp Tyr Asp Ser Ala Ile Arg
    50                  55                  60


Ser Arg Leu Ser Ile Thr Arg Asp Thr Ser Lys Ser Gln Val Phe Leu
65                  70                  75                  80


Lys Ile Asn Ser Leu Gln Thr Glu Asp Thr Ala Met Tyr Phe Cys Ala
                85                  90                  95


Arg Tyr Tyr Phe Ser Ser Tyr Gly Gly Gly Tyr Phe Asp Tyr Trp Gly
            100                 105                 110


Gln Gly Val Met Val Thr Val Ser Ser
        115                 120


<210>  22
<211>  363
<212>  DNA
<213>  Artificial Sequence

<400>  22
caagtccaac taaaggagtc aggacctggt ctggtacagc catcacagac cctgtctctc      60

acctgcactg tctctgggtt ttcattaacc agccatactg taagctggat tcggcagcct 120

ccaggaaggg gtctggagtg gatgggaaaa atgtggagta atggagacac agattatgat 180

tcagctatca gatcccgact gagcatcacc agggacacct cgaagagcca agttttctta 240

aagataaaca gtctgcaaac tgaagacaca gccatgtact tctgtgccag atattacttt 300

agcagctatg gaggtggcta ctttgattac tggggccaag gagtcatggt cacagtctcc 360

tca 363

<210> 23
<211> 106
<212> PRT
<213> Artificial Sequence

<400> 23

Asp Thr Val Leu Thr Gln Ser Pro Ala Leu Ala Val Ser Leu Gly Gln
1               5                   10                  15

Arg Val Thr Ile Ser Cys Arg Ala Ser Lys Ser Val Ser Thr Tyr Met
            20                  25                  30

His Trp Tyr Gln Gln Lys Ser Gly Gln Gln Pro Lys Leu Leu Ile Tyr
        35                  40                  45

Ser Ala Ser Asn Leu Glu Ser Gly Val Pro Ser Arg Phe Ser Gly Ser
    50                  55                  60

Gly Ser Gly Thr Asp Phe Thr Leu Thr Ile Asp Pro Val Glu Ala Asp
65                  70                  75                  80

Asp Ile Ala Asn Tyr Tyr Cys Gln Gln Ser Asp Glu Leu Pro Asp Thr
                85                  90                  95

Phe Gly Ala Gly Thr Lys Leu Glu Leu Lys
            100                 105

<210> 24
<211> 318
<212> DNA
<213> Artificial Sequence

<400> 24
gacactgtgc tgacccagtc tcctgctttg gctgtgtctc taggacagag ggtcaccatc 60

tcttgtaggg ccagcaaaag tgtcagtaca tatatgcact ggtaccaaca gaaatcggga 120

cagcaaccca aactcctgat ctatagtgca tccaacctag aatctggagt cccttccagg 180

ttcagtggga gtgggtctgg gacagacttt accctcacca tagatcctgt ggaggctgat 240

gacatagcaa actattactg tcagcagagt gatgaacttc cggacacctt tggagctggg 300

accaagctgg aactgaaa                                                    318

<210>  25
<211>  119
<212>  PRT
<213>  Artificial Sequence

<400>  25

Glu Val Gln Leu Leu Glu Ser Gly Gly Gly Leu Val Gln Pro Gly Arg
1               5                   10                  15

Ser Met Lys Leu Ser Cys Ala Ala Ser Gly Phe Thr Phe Ser Ser Phe
            20                  25                  30

Pro Met Ala Trp Val Arg Gln Thr Pro Ser Lys Ala Leu Glu Trp Val
        35                  40                  45

Ala Thr Ile Ser Pro Ser Gly Gly Thr Ser Tyr Ser Arg Asp Ser Val
    50                  55                  60

Lys Gly Arg Phe Thr Ile Ser Arg Asp Asn Ala Asn Ser Thr Leu Cys
65                  70                  75                  80

Leu His Met Asp Ser Leu Lys Ser Glu Asp Thr Ala Thr Tyr Tyr Cys
                85                  90                  95

Leu Arg Glu Arg Ile Tyr Asn Thr Tyr Phe Asp Tyr Trp Gly Gln Gly
            100                 105                 110

Val Met Val Thr Val Ser Ser
        115

<210>  26
<211>  357
<212>  DNA
<213>  Artificial Sequence

<400>  26
gaggtgcagc tgctagaatc tgggggcggt ttagtgcagc ctggaaggtc catgaaactc      60

tcctgtgcgg cctcaggatt cactttcagt agctttccaa tggcctgggt ccgccagact     120

ccatcgaagg ctctggagtg ggtcgcaacc attagtccta gtggtggtac ctcttactct     180

cgagactccg tgaagggccg attcactatc tccagagata atgcaaacag caccctatgt     240

ctacacatgg acagtctgaa gtctgaggac acggccactt attattgttt acgagaaagg     300

atttataaca cttactttga ttactggggc caaggagtca tggtcacagt ctcctca        357

<210>  27

<211>  107
<212>  PRT
<213>  Artificial Sequence

<400>  27

Asp Ile Arg Met Thr His Ser Pro Ala Ser Leu Ser Ala Ser Leu Gly
1               5                   10                  15

Glu Thr Val Ser Ile Glu Cys Leu Pro Ser Glu Asp Ile Ser Ser Asp
            20                  25                  30

Leu Ala Trp Tyr Gln Gln Lys Pro Gly Lys Ser Pro Gln Leu Leu Ile
        35                  40                  45

Tyr Asn Ala Asn Thr Leu Pro Asn Gly Val Pro Ser Arg Phe Ser Gly
    50                  55                  60

Ser Arg Ser Gly Ala Gln Tyr Phe Leu Lys Ile Asn Ser Leu Gln Ser
65                  70                  75                  80

Glu Asp Val Ala Thr Tyr Phe Cys Gln Gln Tyr Asp Ser Tyr Pro Tyr
                85                  90                  95

Thr Phe Gly Thr Gly Thr Lys Leu Glu Leu Lys
            100                 105

<210>  28
<211>  321
<212>  DNA
<213>  Artificial Sequence

<400>  28
gatatccgga tgacacactc tccagcttcc ctgtctgcat ctctgggaga gactgtcagc      60

atcgaatgtc taccaagtga ggacatttcc agtgatttag catggtatca gcagaagcca     120

gggaaatctc ctcagctcct catctataat gcaaatactt tgccaaatgg ggtcccttca     180

cggtttagtg gcagtagatc tggcgcacag tattttctaa aaataaacag cctgcaatct     240

gaggatgtcg cgacttattt ctgtcagcaa tatgacagtt atccgtacac gtttggaact     300

gggaccaagc tggaactgaa a                                               321

<210>  29
<211>  5
<212>  PRT
<213>  Artificial Sequence

<220>
<223>  HCDR1

<400>  29

Arg Tyr Trp Met His
1               5

<210>  30
<211>  17
<212>  PRT
<213>  Artificial Sequence

<220>
<223>  HCDR2

<400>  30

Tyr Ile Asn Pro Ile Thr Gly Tyr Thr Glu Asn Asn Gln Lys Phe Lys
1               5                   10                  15

Asp

<210>  31
<211>  7
<212>  PRT
<213>  Artificial Sequence

<220>
<223>  HCDR3

<400>  31

Gly Ile Arg Gly Phe Thr Tyr
1               5

<210>  32
<211>  15
<212>  PRT
<213>  Artificial Sequence

<220>
<223>  LCDR1

<400>  32

Arg Ala Ser Glu Asn Val Asp Asn Ser Asp Asn Ser Phe Met His
1               5                   10                  15

<210>  33
<211>  7
<212>  PRT
<213>  Artificial Sequence

<220>
<223>  LCDR2

<400>  33

Arg Ala Ser Asn Leu Asp Ser
1               5

<210>  34
<211>  9
<212>  PRT
<213>  Artificial Sequence

<220>
<223>  LCDR3

<400>  34

Gln Gln Thr Asn Glu Ala Pro Leu Thr
1               5


<210>  35
<211>  5
<212>  PRT
<213>  Artificial Sequence

<220>
<223>  HCDR1

<400>  35

Asn Tyr Trp Met His
1               5


<210>  36
<211>  17
<212>  PRT
<213>  Artificial Sequence

<220>
<223>  HCDR2

<400>  36

Tyr Val Ile Pro Ser Thr Gly Tyr Thr Asp Tyr Asn Gln Ser Phe Lys
1               5                   10                  15


Asp


<210>  37
<211>  7
<212>  PRT
<213>  Artificial Sequence

<220>
<223>  HCDR3

<400>  37

Leu Leu Pro Gly Phe Ala Tyr
1               5


<210>  38
<211>  16

<212>  PRT
<213>  Artificial Sequence

<220>
<223>  LCDR1

<400>  38

Arg Ser Ser Gln Ser Leu Val Asp Ser Asn Gly Asn Thr Tyr Leu His
1               5                   10                  15


<210>  39
<211>  7
<212>  PRT
<213>  Artificial Sequence

<220>
<223>  LCDR2

<400>  39

Lys Val Ser Asn Arg Phe Ser
1               5


<210>  40
<211>  9
<212>  PRT
<213>  Artificial Sequence

<220>
<223>  LCDR3

<400>  40

Ser Gln Ser Thr His Val Pro Pro Thr
1               5


<210>  41
<211>  17
<212>  PRT
<213>  Artificial Sequence

<220>
<223>  HCDR2

<400>  41

Tyr Ile Asp Pro Arg Thr Ala Ser Ile Tyr Tyr Asn Gln Lys Phe Lys
1               5                   10                  15


Asp


<210>  42
<211>  8
<212>  PRT
<213>  Artificial Sequence

<220>
<223>  HCDR3

<400>  42

Ile Leu Tyr Gly Lys Tyr Asp Val
1               5


<210>  43
<211>  5
<212>  PRT
<213>  Artificial Sequence

<220>
<223>  HCDR1

<400>  43

Asp Ala Trp Met Asp
1               5


<210>  44
<211>  19
<212>  PRT
<213>  Artificial Sequence

<220>
<223>  HCDR2

<400>  44

Glu Ile Arg Ser Lys Thr Tyr His Pro Ala Thr Tyr Tyr Thr Lys Ser
1               5                   10                  15


Val Arg Gly




<210>  45
<211>  10
<212>  PRT
<213>  Artificial Sequence

<220>
<223>  HCDR3

<400>  45

Pro Arg Tyr Tyr Gly Gly Tyr Phe Asp Tyr
1               5                   10


<210>  46
<211>  15
<212>  PRT
<213>  Artificial Sequence

<220>
<223>  LCDR1

<400>  46

Arg Ala Ser Glu Ser Val Asp Asn Tyr Gly Met Ser Phe Met Asn
1               5                   10                  15


<210>  47
<211>  7
<212>  PRT
<213>  Artificial Sequence

<220>
<223>  LCDR2

<400>  47

Thr Ala Ser Asn Gln Gly Ser
1               5


<210>  48
<211>  9
<212>  PRT
<213>  Artificial Sequence

<220>
<223>  LCDR3

<400>  48

Gln Gln Ser Lys Glu Val Pro Tyr Thr
1               5


<210>  49
<211>  5
<212>  PRT
<213>  Artificial Sequence

<220>
<223>  HCDR1

<400>  49

Asn Tyr Ile Ile His
1               5


<210>  50
<211>  17
<212>  PRT
<213>  Artificial Sequence

<220>
<223>  HCDR2

<400>  50

Ala Ile Tyr Pro Gly Asn Gly Asp Thr Ser Tyr Ser Gln Lys Phe Lys
1               5                   10                  15


Asp

<210>  51
<211>  11
<212>  PRT
<213>  Artificial Sequence

<220>
<223>  HCDR3

<400>  51

Gly Asp Ala Gly Tyr Ser Ala Trp Phe Ala Tyr
1               5                   10


<210>  52
<211>  15
<212>  PRT
<213>  Artificial Sequence

<220>
<223>  LCDR1

<400>  52

Ser Ala Ser Glu Ser Val Asp Ser Tyr Gly Asn Asn Phe Met His
1               5                   10                  15


<210>  53
<211>  7
<212>  PRT
<213>  Artificial Sequence

<220>
<223>  LCDR2

<400>  53

Leu Ala Ser Lys Leu Glu Ser
1               5


<210>  54
<211>  9
<212>  PRT
<213>  Artificial Sequence

<220>
<223>  LCDR3

<400>  54

Gln Gln Asn Asn Glu Asp Pro Leu Thr
1               5


<210>  55
<211>  5
<212>  PRT
<213>  Artificial Sequence

<220>
<223>  HCDR1

<400>  55

Ser His Thr Val Ser
1               5


<210>  56
<211>  16
<212>  PRT
<213>  Artificial Sequence

<220>
<223>  HCDR2

<400>  56

Lys Met Trp Ser Asn Gly Asp Thr Asp Tyr Asp Ser Ala Ile Arg Ser
1               5                   10                  15


<210>  57
<211>  13
<212>  PRT
<213>  Artificial Sequence

<220>
<223>  HCDR3

<400>  57

Tyr Tyr Phe Ser Ser Tyr Gly Gly Gly Tyr Phe Asp Tyr
1               5                   10


<210>  58
<211>  11
<212>  PRT
<213>  Artificial Sequence

<220>
<223>  LCDR1

<400>  58

Arg Ala Ser Lys Ser Val Ser Thr Tyr Met His
1               5                   10


<210>  59
<211>  7
<212>  PRT
<213>  Artificial Sequence

<220>
<223>  LCDR2

<400>  59

Ser Ala Ser Asn Leu Glu Ser

1               5


<210>  60
<211>  9
<212>  PRT
<213>  Artificial Sequence

<220>
<223>  LCDR3

<400>  60

Gln Gln Ser Asp Glu Leu Pro Asp Thr
1               5


<210>  61
<211>  5
<212>  PRT
<213>  Artificial Sequence

<220>
<223>  HCDR1

<400>  61

Ser Phe Pro Met Ala
1               5


<210>  62
<211>  17
<212>  PRT
<213>  Artificial Sequence

<220>
<223>  HCDR2

<400>  62

Thr Ile Ser Pro Ser Gly Gly Thr Ser Tyr Ser Arg Asp Ser Val Lys
1               5                   10                  15


Gly



<210>  63
<211>  10
<212>  PRT
<213>  Artificial Sequence

<220>
<223>  HCDR3

<400>  63

Glu Arg Ile Tyr Asn Thr Tyr Phe Asp Tyr
1               5                   10

<210>  64
<211>  11
<212>  PRT
<213>  Artificial Sequence

<220>
<223>  LCDR1

<400>  64

Leu Pro Ser Glu Asp Ile Ser Ser Asp Leu Ala
1               5                   10


<210>  65
<211>  7
<212>  PRT
<213>  Artificial Sequence

<220>
<223>  LCDR2

<400>  65

Asn Ala Asn Thr Leu Pro Asn
1               5


<210>  66
<211>  9
<212>  PRT
<213>  Artificial Sequence

<220>
<223>  LCDR3

<400>  66

Gln Gln Tyr Asp Ser Tyr Pro Tyr Thr
1               5